# EUROPEAN PATENT APPLICATION

(11) **EP 4 299 747 A1**
(43) Date of publication of application: **03.01.2024**
(21) Application number: 23747043.0
(22) Date of filing: 26.01.2023
(51) Int. Cl.: C12N 15/55, A61K 35/12, A61K 38/46, A61K 48/00, A61P 43/00, C12N 1/15, C12N 1/19, C12N 1/21, C12N 5/10, C12N 9/16, C12N 15/09

(54) **MUTANT MAD7 PROTEIN**

(30) Priority: 28.01.2022 JP 2022011914
(71) Applicant: Setsurotech Inc., Tokushima-shi, Tokushima, 770-8503 (JP)
(72) Inventor: HOZUMI Shunya, Tokushima-shi, Tokushima 770-8503 (JP); NISHIMURA Koichi, Tokushima-shi, Tokushima 770-8503 (JP); MASUDA Okura, Tokushima-shi, Tokushima 770-8503 (JP); TAKADERA Satoru, Tokushima-shi, Tokushima 770-8503 (JP); KAINUMA Risa, Tokushima-shi, Tokushima 770-8503 (JP); YAMASHITA Yukiko, Tokushima-shi, Tokushima 770-8503 (JP); SAWATSUBASHI Shun, Tokushima-shi, Tokushima 770-8503 (JP)
(74) Representative: J A Kemp LLP
(86) International application number: PCT/JP2023/002475
(87) International publication number: WO 2023/145833

(57) **Abstract**

A mutant MAD7 protein with higher target-site cleavage activity is provided. The mutant MAD7 protein includes an amino acid sequence B obtained by introducing mutations into an amino acid sequence A represented by SEQ ID NO: 1, the amino acid sequence B including amino-acid substitution mutations of K169 and D529 in the amino acid sequence A.

## Description

### BACKGROUND OF THE INVENTION

### Field of the Invention

The present invention relates to a mutant MAD7 protein and the like.

### Description of Related Art

Currently, genetically modified animals are used to elucidate basic biological mechanisms or are used as human disease models in various research fields including medical and biological fields. Systems in which artificial restriction enzymes such as ZFNs (Zinc-Finger Nucleases), TALENs (Transcription Activator-Like Effector Nucleases), and a CRISPR/Cas System (Clustered Regularly Interspaced Short Palindromic Repeat-Associated System) are used have been attracting attention as means for rapidly producing genetically modified animals. These novel techniques are called "genome editing", and enable modification of the genomes of a wide variety of organisms without using embryonic stem cells (ES cells) and induced pluripotent stem cells (iPS cells).

Although a Cas9 protein is widely used as the Cas protein, novel Cas proteins such as an MAD7 protein are also increasingly used. Also, attempts are made to improve the activity of the MAD7 protein by introducing various mutations (WO 2020/086475).

### Citation List

### Patent Literature

[Patent Literature 1]: WO 2020/086475

### SUMMARY OF THE INVENTION

### Technical Problem

It is an object of the present invention to provide a mutant MAD7 protein with higher target-site cleavage activity.

### Solution to Problem

As a result of extensive research conducted in light of the object above, the inventors of the present invention found that the object above can be achieved in the case where a mutant MAD7 protein includes an amino acid sequence B obtained by introducing mutations into an amino acid sequence A represented by SEQ ID NO: 1, and the amino acid sequence B includes amino-acid substitution mutations of K169 and D529 in the amino acid sequence A. The inventors conducted further research based on this finding and thus accomplished the present invention. That is to say, the present invention encompasses the following aspects.
Amino acid sequence of MAD7 protein (SEQ ID NO: 1)

MNNGTNNFQNFIGISSLQKTLRNALIPTETTQQFIVKNGIIKEDELRGENRQILKDIMDDY
YRGFISETLSSIDDIDWTSLFEKMEIQLKNGDNKDTLIKEQTEYRKAIHKKFANDDRFKN
MFSAKLISDILPEFVIHNNNYSASEKEEKTQVIKLFSRFATSFKDYFKNRANCFSADDISSSS
CHRIVNDNAEIFFSNALVYRRIVKSLSNDDINKISGDMKDSLKEMSLEEIYSYEKYGEFITQ
EGISFYNDICGKVNSFMNLYCQKNKENKNLYKLQKLHKQILCIADTSYEVPYKFESDEEV
YQSVNGFLDNISSKHIVERLRKIGDNYNGYNLDKIYIVSKFYESVSQKTYRDWETINTALE
IHYNNILPGNGKSKADKVKKAVKNDLQKSITEINELVSNYKLCSDDNIKAETYIHEISHILN
NFEAQELKYNPEIHLVESELKASELKNVLDVIMNAFHWCSVFMTEELVDKDNNFYAELE
EIYDEIYPVISLYNLVRNYVTQKPYSTKKIKLNFGIPTLADGWSKSKEYSNNAIILMRDNLY
YLGIFNAKNKPDKKIIEGNTSENKGDYKKMIYNLLPGPNKMIPKVFLSSKTGVETYKPSA
YILEGYKQNKHIKSSKDFDITFCHDLIDYFKNCIAIHPEWKNFGFDFSDTSTYEDISGFYRE
VELQGYKIDWTYISEKDIDLLQEKGQLYLFQIYNKDFSKKSTGNDNLHTMYLKNLFSEEN
LKDIVLKLNGEAEIFFRKSSIKNPIIHKKGSILVNRTYEAEEKDQFGNIQIVRKNIPENIYQEL
YKYFNDKSDKELSDEAAKLKNVVGHHEAATNIVKDYRYTYDKYFLHMPITINFKANKT
GFINDRILQYIAKEKDLHVIGIDRGERNLIYVSVIDTCGNIVEQKSFNIVNGYDYQIKLKQQ
EGARQIARKEWKEIGKIKEIKEGYLSLVIHEISKMVIKYNAIIAMEDLSYGFKKGRFKVER
QVYQKFETMLINKLNYLVFKDISITENGGLLKGYQLTYIPDKLKNVGHQCGCIFYVPAAY
TSKIDPTTGFVNIFKFKDLTVDAKREFIKKFDSIRYDSEKNLFCFTFDYNNFITQNTVMSKS
SWSVYTYGVRIKRRFVNGRFSNESDTIDITKDMEKTLEMTDINWRDGHDLRQDIIDYEIV
QHIFEIFRLTVQMRNSLSELEDRDYDRLISPVLNENNIFYDSAKAGDALPKDADANGAYCI
ALKGLYEIKQITENWKEDGKFSRDKLKISNKDWFDFIQNKRYL

Item 1: A mutant MAD7 protein comprising an amino acid sequence B obtained by introducing mutations into an amino acid sequence A represented by SEQ ID NO: 1, the amino acid sequence B including amino-acid substitution mutations of K169 and D529 in the amino acid sequence A.

Item 2: The mutant MAD7 protein according to item 1, in which the amino acid sequence B includes an amino acid sequence of (a1), (a2), or (a3) below:
(a1) an amino acid sequence that includes amino-acid substitution mutations of K169 and D529 in the amino acid sequence represented by SEQ ID NO: 1;
(a2) an amino acid sequence that is obtained by performing deletion, insertion, substitution, and/or addition of one or several amino acids on the amino acid sequence represented by SEQ ID NO:1 and includes the amino-acid substitution mutations of (a1) above; and
(a3) an amino acid sequence that has 70% or more identity to the amino acid sequence represented by SEQ ID NO: 1 and includes the amino-acid substitution mutations of (a1) above.

Item 3: The mutant MAD7 protein according to item 1 or 2, in which the amino-acid substitution mutations are substitution of K169 with a basic amino acid other than lysine, and substitution of D529 with a basic amino acid.

Item 4: The mutant MAD7 protein according to any one of items 1 to 3, in which the amino-acid substitution mutations are substitution of K169 with arginine, and substitution of D529 with arginine.

Item 5: The mutant MAD7 protein according to any one of items 1 to 4, in which the amino acid sequence B includes an amino-acid substitution mutation of Y1086 in the amino acid sequence A.

Item 6: The mutant MAD7 protein according to item 5, in which the amino-acid substitution mutation is substitution of Y1086 with an aromatic amino acid other than tyrosine.

Item 7: The mutant MAD7 protein according to item 5 or 6, in which the amino-acid substitution mutation is substitution of Y1086 with phenylalanine.

Item 8: The mutant MAD7 protein according to any one of items 1 to 7, in which the amino acid sequence B includes an amino-acid substitution mutation of at least one amino acid selected from the group consisting of K970 and E1227 in the amino acid sequence A.

Item 9: The mutant MAD7 protein according to item 8, in which the amino-acid substitution mutation is a substitution of K970 with a hydrophilic neutral amino acid and/or a substitution of E1227 with a basic amino acid.

Item 10: The mutant MAD7 protein according to item 8 or 9, in which the amino-acid substitution mutation is a substitution of K970 with asparagine and/or a substitution of E1227 with lysine.

Item 11: The mutant MAD7 protein according to any one of items 1 to 10, in which the amino acid sequence B includes an amino-acid substitution mutation of at least one amino acid selected from the group consisting of M961, K1098, F1198, Q1230, I1231, and N1250 in the amino acid sequence A.

Item 12: The mutant MAD7 protein according to any one of items 1 to 11, in which the amino acid sequence B includes an amino-acid substitution mutation of at least one amino acid selected from the group consisting of C892, Y907, I922, Y966, S1000, T1014, K1040, L1056, I1065, K1067, T1083, F1163, D1200, K1236, D1238, F1241, S1242, andK1247 in the amino acid sequence A.

Item 13: The mutant MAD7 protein according to any one of items 1 to 12, in which the amino acid sequence B includes an amino acid sequence of (b1), (b2), or (b3) below:
(b1) an amino acid sequence represented by SEQ ID NO:2;
(b2) an amino acid sequence that is obtained by performing deletion, insertion, substitution, and/or addition of one or several amino acids on the amino acid sequence represented by SEQ ID NO:2 and in which amino acids at position 169 and position 529 in the amino acid sequence represented by SEQ ID NO:2 are conserved; and
(b3) an amino acid sequence that has 70% or more identity to the amino acid sequence represented by SEQ ID NO:2 and in which amino acids at position 169 and position 529 in the amino acid sequence represented by SEQ ID NO:2 are conserved.

Item 14: The mutant MAD7 protein according to item 13, in which, in (b2) or (b3) above, an amino acid at position 1086 in the amino acid sequence represented by SEQ ID NO:2 is further conserved.

Item 15: The mutant MAD7 protein according to item 13 or 14, in which, in (b2) or (b3) above, an amino acid at position 970 and/or an amino acid at position 1227 in the amino acid sequence represented by SEQ ID NO:2 are further conserved.

Item 16: The mutant MAD7 protein according to any one of items 1 to 12, in which the amino acid sequence B includes an amino acid sequence of (c1), (c2), or (c3) below:
(c1) an amino acid sequence represented by SEQ ID NO:3;
(c2) an amino acid sequence that is obtained by performing deletion, insertion, substitution, and/or addition of one or several amino acids on the amino acid sequence represented by SEQ ID NO:3 and in which amino acids at position 169 and position 529 in the amino acid sequence represented by SEQ ID NO:3 are conserved; and
(c3) an amino acid sequence that has 70% or more identity to the amino acid sequence represented by SEQ ID NO:3 and in which amino acids at position 169 and position 529 in the amino acid sequence represented by SEQ ID NO:3 are conserved.

Item 17: The mutant MAD7 protein according to item 13 or 14, in which, in (c2) or (c3) above, an amino acid at position 1086 in the amino acid sequence represented by SEQ ID NO:3 is further conserved.

Item 18: The mutant MAD7 protein according to item 16 or 17, in which, in (c2) or (c3) above, an amino acid at position 970 and/or an amino acid at position 1227 in the amino acid sequence represented by SEQ ID NO:3 are further conserved.

Item 19: The mutant MAD7 protein according to any one of items 1 to 18, including the amino acid sequence B and a nuclear localization signal sequence.

Item 20: A combination of polypeptides, including a first split fragment and a second split fragment of the mutant MAD7 protein according to any one of items 1 to 18,
in which the first split fragment and the second split fragment are polypeptides having amino acid sequences selected from the group consisting of (1) to (10) and (11) below:
(1) the first split fragment has an amino acid sequence corresponding to an amino acid sequence between positions 1 and 182 in SEQ ID NO: 1, and the second split fragment has an amino acid sequence corresponding to an amino acid sequence between positions 183 and 1263 in SEQ ID NO:1;
(2) the first split fragment has an amino acid sequence corresponding to an amino acid sequence between positions 1 and 273 in SEQ ID NO: 1, and the second split fragment has an amino acid sequence corresponding to an amino acid sequence between positions 274 and 1263 in SEQ ID NO:1;
(3) the first split fragment has an amino acid sequence corresponding to an amino acid sequence between positions 1 and 292 in SEQ ID NO: 1, and the second split fragment has an amino acid sequence corresponding to an amino acid sequence between positions 293 and 1263 in SEQ ID NO:1;
(4) the first split fragment has an amino acid sequence corresponding to an amino acid sequence between positions 1 and 377 in SEQ ID NO: 1, and the second split fragment has an amino acid sequence corresponding to an amino acid sequence between positions 378 and 1263 in SEQ ID NO:1;
(5) the first split fragment has an amino acid sequence corresponding to an amino acid sequence between positions 1 and 511 in SEQ ID NO: 1, and the second split fragment has an amino acid sequence corresponding to an amino acid sequence between positions 512 and 1263 in SEQ ID NO:1;
(6) the first split fragment has an amino acid sequence corresponding to an amino acid sequence between positions 1 and 538 in SEQ ID NO: 1, and the second split fragment has an amino acid sequence corresponding to an amino acid sequence between positions 539 and 1263 in SEQ ID NO:1;
(7) the first split fragment has an amino acid sequence corresponding to an amino acid sequence between positions 1 and 567 in SEQ ID NO: 1, and the second split fragment has an amino acid sequence corresponding to an amino acid sequence between positions 568 and 1263 in SEQ ID NO:1;
(8) the first split fragment has an amino acid sequence corresponding to an amino acid sequence between positions 1 and 755 in SEQ ID NO: 1, and the second split fragment has an amino acid sequence corresponding to an amino acid sequence between positions 756 and 1263 in SEQ ID NO:1;
(9) the first split fragment has an amino acid sequence corresponding to an amino acid sequence between positions 1 and 832 in SEQ ID NO: 1, and the second split fragment has an amino acid sequence corresponding to an amino acid sequence between positions 833 and 1263 in SEQ ID NO:1;
(10) the first split fragment has an amino acid sequence corresponding to an amino acid sequence between positions 1 and 854 in SEQ ID NO: 1, and the second split fragment has an amino acid sequence corresponding to an amino acid sequence between positions 855 and 1263 in SEQ ID NO: 1; and
(11) the first split fragment has an amino acid sequence corresponding to an amino acid sequence between positions 1 and 49 and between positions 512 and 1263 in SEQ ID NO: 1 in which an amino acid Y corresponding to position 513 in SEQ ID NO: 1 is substituted with S, and the second split fragment has an amino acid sequence corresponding to an amino acid sequence between positions 50 and 511 in SEQ ID NO: 1.

Item 21: A polynucleotide including a coding sequence of the mutant MAD7 protein according to any one of items 1 to 19 and/or coding sequences of the polypeptides according to item 20.

Item 22: A vector including the polynucleotide according to item 21.

Item 23: A composition including the mutant MAD7 protein according to any one of items 1 to 19, the combination of polypeptides according to item 20, the polynucleotide according to item 21, and/or the vector according to item 22, and a guide RNA targeted on a target site.

Item 24: A cell including the mutant MAD7 protein according to any one of items 1 to 19, the combination of polypeptides according to item 20, the polynucleotide according to item 21, and/or the vector according to item 22.

Item 25: A genome editing method including introducing, into a cell or non-human organism, the mutant MAD7 protein according to any one of items 1 to 19, the combination of polypeptides according to item 20, the polynucleotide according to item 21, and/or the vector according to item 22.

Item 26: The genome editing method according to item 25, further including introducing a guide RNA targeted on a target site into the cell or non-human organism.

Item 27: A method for producing a cell or organism in which a target site is genome-edited, including
introducing into a cell or non-human organism, the mutant MAD7 protein according to any one of items 1 to 19, the combination of polypeptides according to item 20, the polynucleotide according to item 21, and/or the vector according to item 22, and the guide RNA targeted on a target site.

Item 28: A cell or organism obtained using the production method according to item 27.

Item 29: A genome editing composition for experimental use including the mutant MAD7 protein according to any one of items 1 to 19, the combination of polypeptides according to item 20, the polynucleotide according to item 21, the vector according to item 22, and/or the cell according to item 24.

Item 30: A genome editing composition for agricultural use including the mutant MAD7 protein according to any one of items 1 to 19, the combination of polypeptides according to item 20, the polynucleotide according to item 21, the vector according to item 22, and/or the cell according to item 24.

Item 31: A genome editing composition for medical use including the mutant MAD7 protein according to any one of items 1 to 19, the combination of polypeptides according to item 20, the polynucleotide according to item 21, the vector according to item 22, and/or the cell according to item 24.

Item 32: A genome editing composition for livestock use including the mutant MAD7 protein according to any one of items 1 to 19, the combination of polypeptides according to item 20, the polynucleotide according to item 21, the vector according to item 22, and/or the cell according to item 24.

Item 3 3: A genome editing composition for fishery use including the mutant MAD7 protein according to any one of items 1 to 19, the combination of polypeptides according to item 20, the polynucleotide according to item 21, the vector according to item 22, and/or the cell according to item 24.

Item 34: A genome editing composition for industry use including the mutant MAD7 protein according to any one of items 1 to 19, the combination of polypeptides according to item 20, the polynucleotide according to item 21, the vector according to item 22, and/or the cell according to item 24.

### Advantageous Effects of Invention

With the present invention, it is possible to provide a mutant MAD7 protein with higher target-site cleavage activity. In the case where this mutant MAD7 protein is used, genome editing can be performed with a higher degree of efficiency as compared with the case where a wild-type MAD7 protein is used.

### BRIEF DESCRIPTION OF THE DRAWINGS

FIG. 1 shows schematic diagrams illustrating the domains and mutation sites of ST7, ST8-1, and ST8-2.
FIG. 2 shows the amino acid sequence of ST7 (SEQ ID NO:8).
FIG. 3 shows the amino acid sequence of ST8-1 (SEQ ID NO:9).
FIG. 4 shows the amino acid sequence of ST8-2 (SEQ ID NO: 10).
FIG. 5 shows the results of EGxxFP assay in Test Example 2. The vertical axis indicates relative values of the EGFP values normalized by the RFP values (the higher the value is, the higher the cleavage activity is). The horizontal axis indicates the MAD7 expression vectors used. See FIG. 1 for ST7, ST8-1, and ST8-2. The employed PAM sequence is shown on the upper side of the graph.
FIG. 6 shows the results of EGxxFP assay in Test Example 2. The description of this diagram is the same as that of FIG. 5.
FIG. 7 shows the results of EGxxFP assay in Test Example 2. The description of this diagram is the same as that of FIG. 5.
FIG. 8 shows the results of EGxxFP assay in Test Example 2. The description of this diagram is the same as that of FIG. 5.
FIG. 9 shows the results of EGxxFP assay in Test Example 2. The description of this diagram is the same as that of FIG. 5.
FIG. 10 shows the results of EGxxFP assay in Test Example 2. The description of this diagram is the same as that of FIG. 5.
FIG. 11 shows the results of EGxxFP assay in Test Example 2. The description of this diagram is the same as that of FIG. 5.
FIG. 12 shows the results of EGxxFP assay in Test Example 2. The description of this diagram is the same as that of FIG. 5.
FIG. 13 shows the results of EGxxFP assay in Test Example 2. The description of this diagram is the same as that of FIG. 5.
FIG. 14 shows the results of EGxxFP assay in Test Example 2. The description of this diagram is the same as that of FIG. 5.
FIG. 15 shows the results of EGxxFP assay in Test Example 2. The description of this diagram is the same as that of FIG. 5.
FIG. 16 shows the results of EGxxFP assay in Test Example 2. The description of this diagram is the same as that of FIG. 5.
FIG. 17 shows the results of EGxxFP assay in Test Example 2. The description of this diagram is the same as that of FIG. 5.
FIG. 18 shows the results of EGxxFP assay in Test Example 2. The description of this diagram is the same as that of FIG. 5.
FIG. 19 shows the results of EGxxFP assay in Test Example 2. The description of this diagram is the same as that of FIG. 5. The items other than ST7 indicated by the horizontal axis are the mutation sites of the mutant MAD7 proteins coded for by MAD7 expression vectors in SEQ ID NO:1.
FIG. 20 shows the results of EGxxFP assay in Test Example 2. The description of this diagram is the same as that of FIG. 19.
FIG. 21 shows the results of EGxxFP assay in Test Example 2. The description of this diagram is the same as that of FIG. 19.
FIG. 22 shows the results of EGxxFP assay in Test Example 2. The description of this diagram is the same as that of FIG. 19.
FIG. 23 shows the results of EGxxFP assay in Test Example 2. The description of this diagram is the same as that of FIG. 19.
FIG. 24 shows the results of EGxxFP assay in Test Example 2. The description of this diagram is the same as that of FIG. 19.
FIG. 25 shows the results of EGxxFP assay in Test Example 2. The description of this diagram is the same as that of FIG. 19.
FIG. 26 shows the results of in vitro assay in Test Example 4. The vertical axis indicates the cleavage activity. The horizontal axis indicates the proteins used to measure the activity and the measurement temperatures. The employed PAM sequence and the reaction time are shown on the upper side of the graph.
FIG. 27 shows the results of in vitro assay in Test Example 4. The description of this diagram is the same as that of FIG. 26.
FIG. 28 shows the results of in vitro assay in Test Example 4. The description of this diagram is the same as that of FIG. 26.
FIG. 29 shows the results of in vitro assay in Test Example 4. The description of this diagram is the same as that of FIG. 26.
FIG. 30 shows the results of analysis of mutagenesis efficiency using a mouse fertilized egg in Test Example 5. The vertical axis indicates the mutagenesis efficiency, and the horizontal axis indicates the proteins introduced into mouse fertilized eggs.

### DETAILED DESCRIPTION OF THE INVENTION

### 1. Definitions, Etc.

The expressions "contain" and "include" as used herein encompass the concepts of "contain", "include", "consist essentially of", and "consist of".

The term "identity" regarding amino acid sequences as used herein refers to a degree of consistency between two or more comparable amino acid sequences. Accordingly, the higher the consistency between two certain amino acid sequences is, the higher the identity or similarity between the sequences is. The level of identity between amino acid sequences is determined, for example, using a sequence analysis tool "FASTA" with default parameters. Alternatively, the level of identity between amino acid sequences can be determined using an algorithm "BLAST" by Karlin and Altschul (Karlin S, Altschul SF. "Methods for assessing the statistical significance of molecular sequence features by using general scoring schemes" Proc Natl Acad Sci USA. 87: 2264-2268 (1990), and Karlin S, Altschul SF. "Applications and statistics for multiple high-scoring segments in molecular sequences." Proc Natl Acad Sci USA. 90: 5873-7 (1993)). A program called "BLASTX" based on such BLAST algorithm has been developed. The specific procedures of these analysis methods are known, and are available on the Website of National Center of Biotechnology Information (NCBI) (http://www.ncbi.nlm.nih.gov/). Also, the "identity" between base sequences is defined in conformity with the description above.

The term "conservative substitution" as used herein means that an amino acid residue is substituted with an amino acid residue having a similar side chain. For example, substitution between amino acid residues having a basic side chain, such as lysine, arginine, and histidine, corresponds to the conservative substitution. In addition, substitution between amino acid residues having an acidic side chain, such as aspartic acid and glutamic acid; substitution between amino acid residues having an uncharged polar side chain, such as glycine, asparagine, glutamine, serine, threonine, tyrosine, and cysteine; substitution between amino acid residues having a non-polar side chain, such as alanine, valine, leucine, isoleucine, proline, phenylalanine, methionine, and tryptophan; substitution between amino acid residues having a β-branched side chain, such as threonine, valine, and isoleucine; and substitution between amino acid residues having an aromatic side chain, such as tyrosine, phenylalanine, tryptophan, and histidine, also correspond to the conservative substitution.

In this specification, nucleotides such as DNA and RNA may be subj ected to known chemical modifications, as illustrated in the following examples. To prevent degradation by hydrolytic enzymes such as nucleases, phosphate residues (phosphates) of each nucleotide can be substituted with chemically modified phosphate residues such as phosphorothioate (PS), methylphosphonate, and phosphorodithionate. A hydroxyl group at position 2 of the sugar (ribose) in each ribonucleotide may be substituted with -OR (where R represents, for example, CH3 (2'-O-Me), CH2CH2OCH3 (2'-O-MOE), CH2CH2NHC(NH)NH2, CH2CONHCH3, CH2CH2CN, and the like). Furthermore, the base moiety (pyrimidine, purine) may be subj ected to chemical modifications, and examples of the chemical modifications include introduction of a methyl group or cationic functional group into position 5 of the pyrimidine base, and substitution of a carbonyl group at position 2 with thiocarbonyl. Moreover, a phosphate moiety or hydroxyl moiety may be subjected to modifications with, for example, biotin, an amino group, a lower alkylamine group, an acetyl group, or the like, but there is no limitation thereto. In addition, BNAs (LNAs), which are nucleotides in which the conformation of the sugar moiety is fixed to the N-type conformation by forming a cross-link between the 2' oxygen and the 4' carbon of the sugar moiety, and the like may also be favorably used.

Specifically, in this specification, an amino-acid mutation is deletion, substitution, insertion, or addition of an amino acid.

In this specification, the position of an amino acid in an amino acid sequence may be indicated by "the single letter code of the amino acid" + "the amino acid number counted from the N-terminal amino acid". For example, "K169" indicates lysine the 169th amino acid from the N-terminus. Moreover, amino-acid substitution mutation may be indicated by "the single letter code of the amino acid prior to mutation" + "the amino acid number counted from the N-terminal amino acid" + "the single letter code of the amino acid after the mutation". For example, "K169R" indicates substitution mutation of lysine the 169th amino acid from the N-terminus with arginine.

### 2. Mutant MAD7 Protein

An aspect of the present invention relates to a mutant MAD7 protein that includes an amino acid sequence B obtained by introducing mutations into an amino acid sequence A represented by SEQ ID NO: 1, the amino acid sequence B including amino-acid substitution mutations of K169 and D529 in the amino acid sequence A (the mutant MAD7 protein may also be referred to as the "mutant MAD7 protein of the present invention" in this specification). The following describes this mutant MAD7 protein.

The amino acid sequence A (SEQ ID NO: 1) is the amino acid sequence of MAD7 protein. The MAD7 protein is Cas 12a protein derived from Eubacterium rectale. The MAD7 protein can be used in the CRISPR/Cas system. For example, the MAD7 protein in the form of a complex with a guide RNA can bind to a target site of a genome DNA and cleave the target site.

The amino acid sequence B is an amino acid sequence obtained by introducing mutations into the amino acid sequence A, and includes, as mutations, amino-acid substitution mutations of K169 and D529 in the amino acid sequence A. The target-site cleavage activity of the amino acid sequence B can be synergistically improved due to the combination of the amino-acid substitution mutations of K169 and D529.

The amino-acid mutation of K169 is preferably substitution with a basic amino acid other than lysine. Examples of the basic amino acid include arginine and histidine, and arginine is particularly preferable.

The amino-acid mutation of D529 is preferably substitution with a basic amino acid. Examples of the basic amino acid include arginine, lysine, and histidine, and arginine is particularly preferable.

Specific examples of the amino acid sequence B include amino acid sequences of (a) below.
(a1) An amino acid sequence that includes amino-acid substitution mutations of K169 and D529 in the amino acid sequence represented by SEQ ID NO: 1;
(a2) an amino acid sequence that is obtained by performing deletion, insertion, substitution, and/or addition of one or several amino acids on the amino acid sequence represented by SEQ ID NO: 1 and includes the amino-acid substitution mutations of (a1) above; and
(a3) an amino acid sequence that has 70% or more identity to the amino acid sequence represented by SEQ ID NO: 1 and includes the amino-acid substitution mutations of (a1) above.

In the description of the amino acid sequence of (a2), the "one or several" amino acids may be, for example, any number of amino acids as long as a protein that includes the amino acid sequence of (a2) has the target-site cleavage activity. The "one or several" amino acids in the description of (a2) above refer to 1 to 378 amino acids, 1 to 315 amino acids, 1 to 252 amino acids, 1 to 189 amino acids, 1 to 126 amino acids, 1 to 63 amino acids, 1 to 50 amino acids, 1 to 37 amino acids, 1 to 25 amino acids, 1 to 12 amino acids, 1 to 6 amino acids, 1 to 3 amino acids, 1 or 2 amino acids, or 1 amino acid, in the amino acid sequence represented by SEQ ID NO: 1. The "target-site cleavage activity" refers to, for example, cleavage activity of a protein in the form of a complex with a guide RNA that can be targeted on a target site (the same applies hereinafter).

In the description of the amino acid sequence of (a3) above, the "identity" may be, for example, any degree of identity as long as a protein that includes the amino acid sequence of (a3) above has the target-site cleavage activity. The "identity" to the amino acid sequence of (a1) above in the description of (a3) above is, for example, 70% or more, 75% or more, 80% or more, 85% or more, 90% or more, 95% or more, 96% or more, 97% or more, 98% or more, or 99% or more.

It can also be said that the amino-acid substitution mutations in the amino acid sequence of (a1) above, namely the amino acid sequence A, are conserved in the amino acid sequences of (a2) and (a3). The amino acid sequences of (a2) and (a3) may also include an amino-acid substitution mutation as described later other than the amino-acid substitution mutations of K169 and D529 in the amino acid sequence A.

It is preferable that the amino acid sequence B further includes, as a mutation, an amino-acid substitution mutation ofY1086 in the amino acid sequence A in addition to the combination of the amino-acid substitution mutations of K169 and D529. The target-site cleavage activity of the amino acid sequence B can be synergistically improved due to the amino-acid substitution mutation of Y1086 being further included.

The amino-acid mutation ofY1086 is preferably substitution with an aromatic amino acid other than tyrosine. Examples of the aromatic amino acid include phenylalanine and tryptophan, and phenylalanine is particularly preferable.

It is preferable that the amino acid sequence B further includes, as a mutation, an amino-acid substitution mutation of at least one amino acid selected from the group consisting of K970 and E1227 in the amino acid sequence A in addition to the combination of the amino-acid substitution mutations of K169 and D529 (particularly, in addition to the combination of the amino-acid substitution mutations of K169, D529, and Y1086). The target-site cleavage activity of the amino acid sequence B can be synergistically improved due to the amino-acid substitution mutation being further included.

The amino-acid mutation of K970 is preferably substitution with a hydrophilic neutral amino acid. Examples of the hydrophilic neutral amino acid include asparagine, glutamine, serine, threonine, tyrosine, and cysteine, and asparagine is particularly preferable.

The amino-acid mutation of E1227 is preferably substitution with a basic amino acid. Examples of the basic amino acid include lysine, arginine, and histidine, and lysine is particularly preferable.

It is preferable that the amino acid sequence B further includes, as a mutation, an amino-acid substitution mutation of at least one amino acid selected from the group consisting of M961, K1098, F1198, Q1230, I1231, and N1250 in the amino acid sequence Ain addition to the combination of the amino-acid substitution mutations of K169 and D529. The target-site cleavage activity of the amino acid sequence B can be improved due to the amino-acid substitution mutation being further included.

The amino-acid mutation of M961 is preferably substitution with a hydrophobic amino acid other than methionine. Examples of the hydrophobic amino acid include leucine, isoleucine, valine, glycine, alanine, proline, tryptophan, and phenylalanine. Leucine, isoleucine, valine, and the like are preferable, and leucine is particularly preferable.

The amino-acid mutation of K1098 is preferably substitution with a hydrophilic neutral amino acid. Examples of the hydrophilic neutral amino acid include asparagine, glutamine, serine, threonine, tyrosine, and cysteine, and asparagine is particularly preferable.

The amino-acid mutation of F1198 is preferably substitution with an aromatic amino acid other than phenylalanine. Examples of the aromatic amino acid include tyrosine and tryptophan, and tyrosine is particularly preferable.

The amino-acid mutation of Q1230 is preferably substitution with a basic amino acid. Examples of the basic amino acid include arginine, lysine, and histidine, and arginine is particularly preferable.

The amino-acid mutation of I1231 is preferably substitution with a hydrophobic amino acid other than isoleucine. Examples of the hydrophobic amino acid include valine, leucine, glycine, alanine, proline, tryptophan, phenylalanine, and methionine, and valine is particularly preferable.

The amino-acid mutation ofN1250 is preferably substitution with a hydrophilic neutral amino acid other than asparagine. Examples of the hydrophilic neutral amino acid include serine, glutamine, threonine, tyrosine, and cysteine, and serine is particularly preferable.

The amino acid sequence B can include other amino-acid mutations (e.g., substitutions or conservative substitutions) as long as the target-site cleavage activity is not significantly impaired. The number of the other amino-acid mutations is, for example, 1 to 50, 1 to 20, 1 to 10, or 1 to 5. Examples of the other amino-acid mutations in the amino acid sequence B include an amino-acid substitution mutation of at least one amino acid selected from the group consisting of C892, Y907, I922, Y966, S1000, T1014, K1040, L1056, I1065,K1067, T1083, F1163, D1200, K1236, D1238, F1241, S1242, and K1247. More specific examples thereof include C892Y, Y907H, I922L, Y966F, S1000G, T1014A, K1040T, L1056M, I1065V, K1067N, T1083A, F1163V, D1200N, K1236R, D1238G, F1241L, S1242R, andK1247R.

The amino acid sequence B may be the amino acid sequence of a split protein fragment of a mutant MAD7 protein (amino acid sequence B') obtained by introducing the amino-acid substitution mutations of K169 and D529 into the amino acid sequence A. The split protein fragments are fragments obtained by cleaving the original protein sequence at one or more sites (e.g., two to three sites, preferably two sites) and can reassemble with each other to restore the original activity.

Splitting sites can be determined based on known information (e.g., Cas9 protein splitting technique). For example, the splitting sites can be determined as follows.
1. A three-dimensional structure is obtained from the amino acid sequence B' through homology modeling.
2. One site per region extending across domains is selected as a cleavage site. At this time, a plurality of model structures is compared, and a candidate that is considered to have no structural disadvantages is preferentially selected.
3. Finally, a site that is considered to be splittable is selected.

If a favorable result cannot be obtained when the protein is cleaved at one site, an attempt should be made to design split proteins based on cleavage at a plurality of sites and reconnection of the resultant fragments. A plurality of patterns is possible for such a design of split proteins. For example, the sequence is split into three regions A/B/C, and then A and C are reconnected, so that the sequence is split into substantially two regions AC and B.

Specific examples include 11 splitting sites and 22 split fragments (heads and tails) as follows. The numbers given in the table are the amino acid numbers in the SEQ ID NO:1.

**Table 1**

| | Amino acid region in domains (aa) | |
|---|---|---|
| | head | tail |
| split182 | 1 to 182 | 183 to 1263 |
| split273 | 1 to 273 | 274 to 1263 |
| split292 | 1 to 292 | 293 to 1263 |
| split377 | 1 to 377 | 378 to 1263 |
| split511 | 1 to 511 | 512 to 1263 |
| split538 | 1 to 538 | 539 to 1263 |
| split567 | 1 to 567 | 568 to 1263 |
| split755 | 1 to 755 | 756 to 1263 |
| split832 | 1 to 832 | 833 to 1263 |
| split854 | 1 to 854 | 855 to 1263 |
| special | 1 to 49, 512 to 1263 (513aa is changed from Y to S) | 50 to 511 |

The amino acid sequence B is preferably an amino acid sequence B1 represented by SEQ ID NO:2 or SEQ ID NO:3, or an amino acid sequence B2 having 80% or more (preferably 85% or more, more preferably 90% or more, even more preferably 95% or more, even more preferably 97% or more, and particularly preferably 99% or more, and less than 100%) identity to the amino acid sequence B1.
Amino acid sequence B1 (amino acid sequence of mutant MAD7 protein (ST8-1s), SEQ ID NO:2) 
MNNGTNNFQNFIGISSLQKTLRNALIPTETTQQFIVKNGIIKEDELRGENRQILKDIMDDY
YRGFISETLSSIDDIDWTSLFEKMEIQLKNGDNKDTLIKEQTEYRKAIHKKFANDDRFKN
MFSAKLISDILPEFVIHNNNYSASEKEEKTQVIKLFSRFATSFKDYFRNRANCFSADDISSSS
CHRIVNDNAEIFFSNALVYRRIVKSLSNDDINKISGDMKDSLKEMSLEEIYSYEKYGEFITQ
EGISFYNDICGKVNSFMNLYCQKNKENKNLYKLQKLHKQILCIADTSYEVPYKFESDEEV
YQSVNGFLDNISSKHIVERLRKIGDNYNGYNLDKIYIVSKFYESVSQKTYRDWETINTALE
IHYNNILPGNGKSKADKVKKAVKNDLQKSITEINELVSNYKLCSDDNIKAETYIHEISHILN
NFEAQELKYNPEIHLVESELKASELKNVLDVIMNAFHWCSVFMTEELVDKDNNFYAELE
EIYDEIYPVISLYNLVRNYVTQKPYSTKKIKLNFGIPTLARGWSKSKEYSNNAIILMRDNLY
YLGIFNAKNKPDKKIIEGNTSENKGDYKKMIYNLLPGPNKMIPKVFLSSKTGVETYKPSA
YILEGYKQNKHIKSSKDFDITFCHDLIDYFKNCIAIHPEWKNFGFDFSDTSTYEDISGFYRE
VELQGYKIDWTYISEKDIDLLQEKGQLYLFQIYNKDFSKKSTGNDNLHTMYLKNLFSEEN
LKDIVLKLNGEAEIFFRKSSIKNPIIHKKGSILVNRTYEAEEKDQFGNIQIVRKNIPENIYQEL
YKYFNDKSDKELSDEAAKLKNVVGHHEAATNIVKDYRYTYDKYFLHMPITINFKANKT
GFINDRILQYIAKEKDLHVIGIDRGERNLIYVSVIDTCGNIVEQKSFNIVNGYDYQIKLKQQ
EGARQIARKEWKEIGKIKEIKEGYLSLVIHEISKMVIKYNAIIAMEDLSYGFKNGRFKVER
QVYQKFETMLINKLNYLVFKDISITENGGLLKGYQLTYIPDKLKNVGHQCGCIFYVPAAY
TSKIDPTTGFVNIFKFKDLTVDAKREFIKKFDSIRYDSEKNLFCFTFDFNNFITQNTVMSKS
SWSVYTYGVRIKRRFVNGRFSNESDTIDITKDMEKTLEMTDINWRDGHDLRQDIIDYEIV
 
QHIFEIFRLTVQMRNSLSELEDRDYDRLISPVLNENNIFYDSAKAGDALPKDADANGAYCI
ALKGLYEIKQITENWKEDGKFSRDKLKISNKDWFDFIQNKRYL
Amino acid sequence B 1 (amino acid sequence of mutant MAD7 protein (ST8-2s), SEQ ID NO:3)

MNNGTNNFQNFIGISSLQKTLRNALIPTETTQQFIVKNGIIKEDELRGENRQILKDIMDDY
YRGFISETLSSIDDIDWTSLFEKMEIQLKNGDNKDTLIKEQTEYRKAIHKKFANDDRFKN
MFSAKLISDILPEFVIHNNNYSASEKEEKTQVIKLFSRFATSFKDYFRNRANCFSADDISSSS
CHRIVNDNAEIFFSNALVYRRIVKSLSNDDINKISGDMKDSLKEMSLEEIYSYEKYGEFITQ
EGISFYNDICGKVNSFMNLYCQKNKENKNLYKLQKLHKQILCIADTSYEVPYKFESDEEV
YQSVNGFLDNISSKHIVERLRKIGDNYNGYNLDKIYIVSKFYESVSQKTYRDWETINTALE
IHYNNILPGNGKSKADKVKKAVKNDLQKSITEINELVSNYKLCSDDNIKAETYIHEISHILN
NFEAQELKYNPEIHLVESELKASELKNVLDVIMNAFHWCSVFMTEELVDKDNNFYAELE
EIYDEIYPVISLYNLVRNYVTQKPYSTKKIKLNFGIPTLARGWSKSKEYSNNAIILMRDNLY
YLGIFNAKNKPDKKIIEGNTSENKGDYKKMIYNLLPGPNKMIPKVFLSSKTGVETYKPSA
YILEGYKQNKHIKSSKDFDITFCHDLIDYFKNCIAIHPEWKNFGFDFSDTSTYEDISGFYRE
VELQGYKIDWTYISEKDIDLLQEKGQLYLFQIYNKDFSKKSTGNDNLHTMYLKNLFSEEN
LKDIVLKLNGEAEIFFRKSSIKNPIIHKKGSILVNRTYEAEEKDQFGNIQIVRKNIPENIYQEL
YKYFNDKSDKELSDEAAKLKNVVGHHEAATNIVKDYRYTYDKYFLHMPITINFKANKT
GFINDRILQYIAKEKDLHVIGIDRGERNLIYVSVIDTCGNIVEQKSFNIVNGYDYQIKLKQQ
EGARQIARKEWKEIGKIKEIKEGYLSLVIHEISKMVIKYNAIIAMEDLSYGFKKGRFKVER
QVYQKFETMLINKLNYLVFKDISITENGGLLKGYQLTYIPDKLKNVGHQCGCIFYVPAAY
TSKIDPTTGFVNIFKFKDLTVDAKREFIKKFDSIRYDSEKNLFCFTFDFNNFITQNTVMSKS
SWSVYTYGVRIKRRFVNGRFSNESDTIDITKDMEKTLEMTDINWRDGHDLRQDIIDYEIV
QHIFEIFRLTVQMRNSLSELEDRDYDRLISPVLNENNIFYDSAKAGDALPKDADANGAYCI
ALKGLYKIKQITENWKEDGKFSRDKLKISNKDWFDFIQNKRYL

The amino acid sequence B may also be an amino acid sequence of (b) below.
(b) An amino acid sequence of (b1), (b2), or (b3) below:
(b 1) the amino acid sequence represented by SEQ ID NO:2;
(b2) an amino acid sequence that is obtained by performing deletion, insertion, substitution, and/or addition of one or several amino acids on the amino acid sequence represented by SEQ ID NO:2 and in which the amino acids at position 169 and position 529 in the amino acid sequence represented by SEQ ID NO:2 are conserved; and
(b3) an amino acid sequence that has 70% or more identity to the amino acid sequence represented by SEQ ID NO:2 and in which the amino acids at position 169 and position 529 in the amino acid sequence represented by SEQ ID NO:2 are conserved.

In the description of the amino acid sequence of (b2), the "one or several" amino acids may be, for example, any number of amino acids as long as a protein that includes the amino acid sequence of (b2) has the target-site cleavage activity. The "one or several" amino acids in the description of (b2) refer to 1 to 378 amino acids, 1 to 315 amino acids, 1 to 252 amino acids, 1 to 189 amino acids, 1 to 126 amino acids, 1 to 63 amino acids, 1 to 50 amino acids, 1 to 37 amino acids, 1 to 25 amino acids, 1 to 12 amino acids, 1 to 6 amino acids, 1 to 3 amino acids, 1 or 2 amino acids, or 1 amino acid, in the amino acid sequence represented by SEQ ID NO:2.

In the description of the amino acid sequence of (b3) above, the "identity" may be, for example, any degree of identity as long as a protein that includes the amino acid sequence of (b3) above has the target-site cleavage activity. The "identity" to the amino acid sequence of (b1) above in the description of (b3) above is, for example, 70% or more, 75% or more, 80% or more, 85% or more, 90% or more, 95% or more, 96% or more, 97% or more, 98% or more, or 99% or more.

In (b2) or (b3), it is preferable that the amino acid at position 1086 in the amino acid sequence represented by SEQ ID NO:2 is further conserved. The target-site cleavage activity of a protein that includes the amino acid sequence of (b2) or (b3) can be synergistically improved due to the amino acid at position 1086 being conserved.

In (b2) or (b3), it is preferable that the amino acid at position 970 and/or the amino acid at position 1227 in the amino acid sequence represented by SEQ ID NO:2 are further conserved. The target-site cleavage activity of a protein that includes the amino acid sequence of (b2) or (b3) can be synergistically improved due to the amino acid at position 970 and/or the amino acid at position 1227 being conserved.

The amino acid sequence B may also be an amino acid sequence of (c) below.
(c) An amino acid sequence of (c1), (c2), or (c3) below:
(c1) the amino acid sequence represented by SEQ ID NO:3;
(c2) an amino acid sequence that is obtained by performing deletion, insertion, substitution, and/or addition of one or several amino acids on the amino acid sequence represented by SEQ ID NO:3 and in which the amino acids at position 169 and position 529 in the amino acid sequence represented by Sequence ID No. 3 are conserved; and
(c3) an amino acid sequence that has 70% or more identity to the amino acid sequence represented by Sequence ID No. 3 and in which the amino acids at position 169 and position 529 in the amino acid sequence represented by Sequence ID No. 3 are conserved.

In the description of the amino acid sequence of (c2), the "one or several" amino acids may be, for example, any number of amino acids as long as a protein that includes the amino acid sequence of (c2) has the target-site cleavage activity. The "one or several" amino acids in the description of (c2) refer to 1 to 378 amino acids, 1 to 315 amino acids, 1 to 252 amino acids, 1 to 189 amino acids, 1 to 126 amino acids, 1 to 63 amino acids, 1 to 50 amino acids, 1 to 37 amino acids, 1 to 25 amino acids, 1 to 12 amino acids, 1 to 6 amino acids, 1 to 3 amino acids, 1 or 2 amino acids, or 1 amino acid, in the amino acid sequence represented by SEQ ID NO:3.

In the description of the amino acid sequence of (c3) above, the "identity" may be, for example, any degree of identity as long as a protein that includes the amino acid sequence of (c3) above has the target-site cleavage activity. The "identity" to the amino acid sequence of (c1) above in the description of (c3) above is, for example, 70% or more, 75% or more, 80% or more, 85% or more, 90% or more, 95% or more, 96% or more, 97% or more, 98% or more, or 99% or more.

In (c2) or (c3), it is preferable that the amino acid at position 1086 in the amino acid sequence represented by SEQ ID NO:3 is further conserved. The target-site cleavage activity of a protein that includes the amino acid sequence of (c2) or (c3) can be synergistically improved due to the amino acid at position 1086 being conserved.

In (c2) or (c3), it is preferable that the amino acid at position 970 and/or the amino acid at position 1227 in the amino acid sequence represented by SEQ ID NO:3 are further conserved. The target-site cleavage activity of a protein that includes the amino acid sequence of (c2) or (c3) can be synergistically improved due to the amino acid at position 970 and/or the amino acid at position 1227 being conserved.

Another amino acid sequence other than the amino acid sequence B may be added to the mutant MAD7 protein of the present invention as long as the target-site cleavage activity is not significantly impaired. Examples of the other amino acid sequence include proteins or peptides such as a nuclear localization signal sequence, a protein tag, a fluorescent protein, a luminescent protein, and a signal sequence such as a protease recognition sequence (e.g., a TEV protease recognition sequence). Examples of the protein tag include biotin, a His tag, a FLAG tag, a Halo tag, an MBP tag, an HA tag, a Myc tag, a V5 tag, and a PA tag.

In one favorable aspect of the present invention, it is preferable that the mutant MAD7 protein of the present invention includes the amino acid sequence B and a nuclear localization signal sequence. Examples of the nuclear localization signal sequence include an SV40 nuclear localization signal sequence and nucleoplasmin nuclear localization signal. When the mutant MAD7 protein of the present invention includes the nuclear localization signal sequence, it is preferable that the nuclear localization signal sequence is disposed on the C-terminal side of the amino acid sequence B.

The mutant MAD7 protein of the present invention may be chemically modified as long as the target-site cleavage activity is not significantly impaired.

The C-terminus of the mutant MAD7 protein of the present invention may be any of a carboxyl group (-COOH), a carboxylate (-COO⁻), an amide (-CONH₂), and an ester (-COOR).

Examples of R in the ester include: C₁₋₆ alkyl groups such as methyl, ethyl, n-propyl, isopropyl, and n-butyl; C₃₋₈ cycloalkyl groups such as cyclopentyl and cyclohexyl; C₆₋₁₂ aryl groups such as phenyl and α-naphthyl; phenyl-C₁₋₂ alkyl groups such as benzyl and phenethyl; C₇₋₁₄ aralkyl groups such as α-naphthyl-C₁₋₂ alkyl groups (e.g., α-naphthylmethyl); and a pivaloyloxymethyl group.

In the mutant MAD7 protein of the present invention, carboxyl groups (or carboxylates) other than the carboxyl group (or carboxylate) at the C-terminus may be amidated or esterified. In this case, the above-mentioned C-terminal esters are used as the esters.

Furthermore, the mutant MAD7 protein of the present invention also encompasses those in which the amino group of the N-terminal amino acid residue is protected by a protecting group (e.g., a C₁₋₆ acyl group such as C₁₋₆ alkanoyl (e.g., a formyl group or acetyl group)), those in which the N-terminal glutamine residue, which may be generated by in-vivo cleavage, becomes pyroglutamate, those in which substituents (e.g., -OH, -SH, amino group, imidazole group, indole group, and guanidino group) on the side chains of amino acids in the molecule are protected by appropriate protecting groups (e.g., a C₁₋₆ acyl group such as a C₁₋₆ alkanoyl group (e.g., a formyl group or acetyl group)), and those in the form of a complex protein such as a sugar chain-bound protein known as a glycoprotein.

The mutant MAD7 protein of the present invention may be in the form of a salt with an acid or base. There is no particular limitation on the salt, and both an acid salt and a basic salt can be employed. Examples of the acid salt include: inorganic acid salts such as hydrochlorides, hydrobromides, sulfates, nitrates, and phosphates; organic acid salts such as acetates, propionates, tartrates, fumarates, maleates, malates, citrates, methanesulfonates, and p-toluenesulfonates; and amino acid salts such as aspartates and glutamates. Examples of the basic salt include: alkali metal salts such as sodium salts and potassium salts; and alkaline-earth metal salts such as calcium salts and magnesium salts.

The mutant MAD7 protein of the present invention may be in the form of a solvate. There is no particular limitation on the solvent, and examples thereof include water, ethanol, glycerol, and acetic acid.

The target-site cleavage activity of the mutant MAD7 protein of the present invention can be measured using a method described in "Examples". The target-site cleavage activity of the mutant MAD7 protein of the present invention is preferably 1.5 or more times, more preferably 2 or more times, even more preferably 3 or more times, and even more preferably 4 or more times as high as the target-site cleavage activity of the MAD7 protein having the amino acid sequence A represented by SEQ ID NO: 1. In these ranges, the activity of the mutant MAD7 protein of the present invention is preferably 5 or more times, more preferably 6 or more times, even more preferably 7 or more times, even more preferably 8 or more times, even more preferably 9 or more times, and particularly preferably 10 or more times as high as the activity of the MAD7 protein having the amino acid sequence A represented by SEQ ID NO: 1. The target-site cleavage activity of the mutant MAD7 protein of the present invention is 10 or less times, 9 or less times, 8 or less times, 7 or less times, 6 or less times, 5 or less times, 4 or less times, 3 or less times, 2 or less times, or 1.5 or less times as high as the target-site cleavage activity of the MAD7 protein having the amino acid sequence A represented by SEQ ID NO: 1. The range of the target-site cleavage activity of the mutant MAD7 protein of the present invention is specified by any combination of the above-described upper limit values and lower limit values.

The mutant MAD7 protein of the present invention can be easily produced in accordance with known genetic engineering techniques. For example, the mutant MAD7 protein of the present invention can be produced using PCR, restriction enzyme digestion, a DNA coupling technique, an in-vitro transcription-translation technique, a recombinant protein production technique, and the like.

### 3. Polynucleotide, Cell

An aspect of the present invention relates to a polynucleotide that includes a coding sequence of the mutant MAD7 protein of the present invention (the polynucleotide may also be referred to as the "polynucleotide of the present invention" in this specification), and a cell that includes at least one selected from the group consisting of the polynucleotide of the present invention and the mutant MAD7 protein of the present invention (the cell may also be referred to as the "cell of the present invention" in this specification). The following describes the polynucleotide and the cell.

There is no particular limitation on the coding sequence of the mutant MAD7 protein of the present invention as long as it is a polynucleotide that includes a base sequence coding for the mutant MAD7 protein of the present invention.

An aspect of the polynucleotide of the present invention includes an expression cassette of the mutant MAD7 protein of the present invention.

There is no particular limitation on the expression cassette of the mutant MAD7 protein of the present invention as long as it is a polynucleotide capable of expressing the mutant MAD7 protein of the present invention in a cell. A typical example of the expression cassette of the mutant MAD7 protein of the present invention is a polynucleotide that includes a promoter and the coding sequence of the mutant MAD7 protein of the present invention disposed under the control of the promoter.

The promoter included in the expression cassette of the mutant MAD7 protein of the present invention is not particularly limited, and can be selected as appropriate depending on target cells. For example, various pol-II promoters can be used as the promoter. There is no particular limitation on the pol-II promoters, but examples thereof include a CMV promoter, an EF1 promoter, an SV40 promoter, and an MSCV promoter. Examples of other promoters include tryptophan promoters such as trc and tac, a lac promoter, a T7 promoter, a T5 promoter, a T3 promoter, an SP6 promoter, an arabinose-induced promoter, a cold-shock promoter, and a tetracycline-induced promoter.

The polynucleotide of the present invention may also include other elements (e.g., a multicloning site (MCS), a drug resistance gene, a replication origin, an enhancer sequence, a repressor sequence, an insulator sequence, a coding sequence of a reporter protein (e.g., fluorescent protein), a coding sequence of a drug resistance gene, and an expression cassette of a guide RNA) as necessary.

The polynucleotide of the present invention can be in the form of a vector. An appropriate vector is selected depending on the intended use (cloning, protein expression, etc.) in consideration of the type of host cell. When Escherichia coli is used as a host, examples of the vector include an M13 phage or modifications thereof, a λ phage or modifications thereof, and pBR322 or modifications thereof (e.g., pB325, pAT153, and pUC8). When yeast is used as a host, examples of the vector include pYepSec1, pMFa, pYES2, and pPIC3.5K. When an insect cell is used as a host, examples of the vector include pAc and pVL. When a mammalian cell is used as a host, examples of the vector include pcDNA, pCDM8, and pMT2PC.

There is no particular limitation on the cell of the present invention as long as it includes at least one selected from the group consisting of the polynucleotide of the present invention and the mutant MAD7 protein of the present invention. Examples of the cell include E. coli such as Escherichia coli K12, bacteria belonging to the genus Bacillus such as Bacillus subtilis MI114, yeast such as Saccharomyces cerevisiae AH22, an Sf cell line derived from Spodoptera frugiperda, a HighFive cell line derived from Trichoplusia ni, insect cells such as an olfactory nerve cell, and animal cells such as a COS7 cell. Favorable examples of the animal cells include cultured cells derived from mammals, and specific examples thereof include a COS7 cell, a CHO cell, a HEK293 cell, a HEK293FT cell, a Hela cell, a PC12 cell, an N1E-115 cell, and an SH-SY5Y cell.

In an aspect of the cell of the present invention that includes the polynucleotide of the present invention, the mutant MAD7 protein of the present invention is expressed from the polynucleotide of the present invention.

### 4. Genome Editing Method

An aspect of the present invention relates to a genome editing method that includes introducing at least one selected from the group consisting of the mutant MAD7 protein of the present invention and the polynucleotide of the present invention into a cell or a non-human organism.

There is no particular limitation on the target cell and organism of genome editing as long as the genome editing can be performed thereon using the CRISPR/Cas system. Examples of the target cell of genome editing include cells derived from various tissues or cells having various properties, such as blood cells, hematopoietic stem cells or precursor cells, gametes (sperms, eggs), fertilized eggs, fibroblasts, epithelial cells, vascular endothelial cells, neurons, hepatocytes, keratin-producing cells, muscle cells, epidermal cells, endocrine cells, ES cells, iPS cells, tissue stem cells, and cancer cells. Examples of the target organism of genome editing include: animals (e.g., mammals such as humans, monkeys, mice, rats, dogs, cats, and rabbits; reptiles such as snakes and lizards; amphibians such as African clawed toads; fishes such as zebrafish, killifish, and tiger puffer; chordates such as ascidians; and arthropods such as fruit flies and silkworms); plants (e.g., thale cress, rice, wheat, and tobacco); algae (e.g., brown seaweed and laver); fungi (e.g., yeast and red bread mold); and bacteria (e.g., E. coli, Bacillus subtilis, and cyanobacteria).

The introduction method is not particularly limited, and can be selected as appropriate depending on the type of target cell or organism, and the type of material (nucleic acid, protein, or the like). Examples of the introduction method include microinjection, electroporation, DEAE-dextran treatment, lipofection, nanoparticle-mediated transfection, and virus-mediated nucleic acid delivery.

Materials that are necessary for genome editing, such as an expression cassette of a guide RNA and a donor DNA, can be introduced as needed.

After a certain period of time has elapsed since the introduction, genome editing starts inside the target cell or target organism, and therefore, this target cell or target organism can be collected to obtain a genome-edited cell or organism.

### 5. Genome Editing Composition

An aspect of the present invention relates to a genome editing composition that includes at least one selected from the group consisting of the mutant MAD7 protein of the present invention, the polynucleotide of the present invention, and the cell of the present invention (the genome editing composition may also be referred to as the "genome editing composition of the present invention" in this specification). The following describes this genome editing composition.

There is no particular limitation on the genome editing composition of the present invention as long as it includes at least one selected from the group consisting of the mutant MAD7 protein of the present invention, the polynucleotide of the present invention, and the cell of the present invention. The genome editing composition may include only these components, or may further include another component as necessary. Examples of the other component include a base, a carrier, a solvent, a dispersant, an emulsifier, a buffer, a stabilizer, a vehicle, a binder, a disintegrator, a lubricant, a thickener, a humectant, a coloring agent, a perfume, and a chelating agent, but there is no particular limitation thereto. The genome editing composition may also include a polynucleotide that includes an expression cassette of a guide RNA as necessary. In addition, the genome editing composition may also include a donor polynucleotide as necessary.

The genome editing composition of the present invention can also be included in a kit. In this case, if necessary, this kit may include other materials, reagents, instruments, and the like that are needed to carry out the genome editing method of the present invention, such as a nucleic acid introducing reagent and a buffer solution, as appropriate. The genome editing kit may also include a polynucleotide that includes an expression cassette of a guide RNA as necessary, as the other material that is needed to carry out the genome editing method of the present invention. In addition, the genome editing kit may also include a donor polynucleotide.

The genome editing composition of the present invention can be used in various applications. The genome editing composition of the present invention can be used for experimental, agricultural, medical, livestock, fishery, and industrial purposes. The experimental use refers to use for testing and research, and not for practical purposes such as agriculture or medicine. The agricultural use encompasses, for example, cases where a genome-edited product is used as an agricultural product. The medical use encompasses, for example, cases in which genome editing is used to improve or treat the condition or disease of an organism. The livestock use encompasses, for example, cases where a genome-edited product is used as a livestock animal. The fishery use encompasses, for example, cases where a genome-edited product is used as a fishery animal. The industrial use encompasses, for example, cases where a genome-edited product is used as an industrial raw material (e.g., an organism that produces materials such as fibers, and a microorganism for fermentation of alcoholic beverages or the like).

### Examples

Hereinafter, the present invention will be described in detail based on examples, but the present invention is not limited to these examples.

### Test Example 1. Preparation of Expression Vector

### Test Example 1-1. Preparation of MAD7 Expression Vector

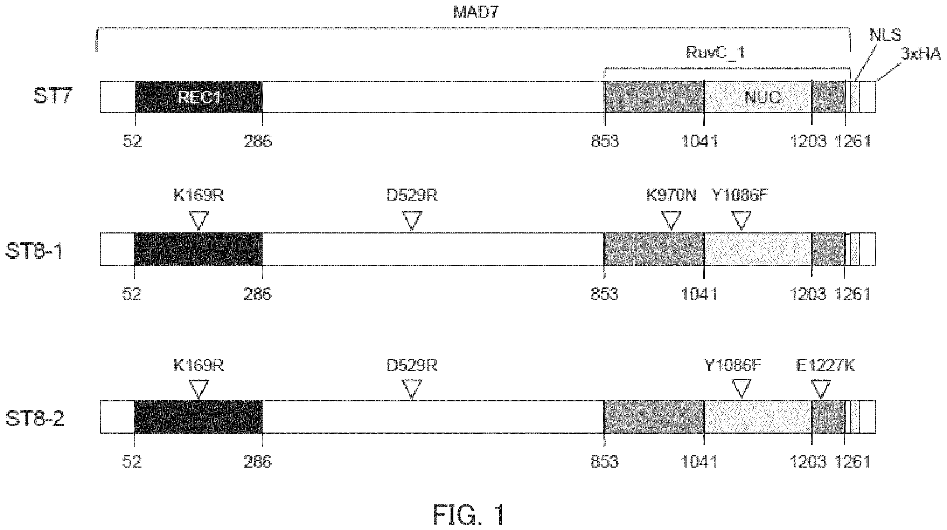

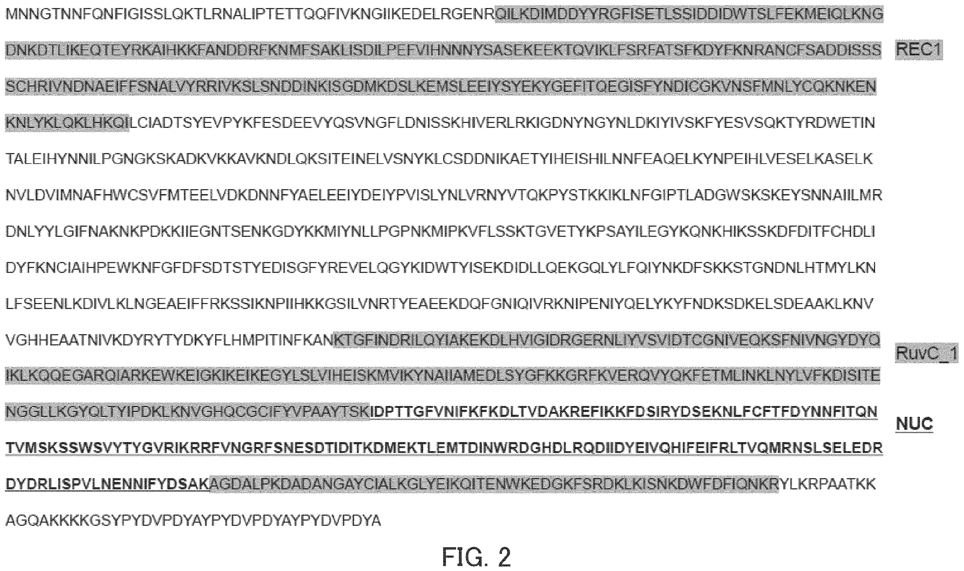

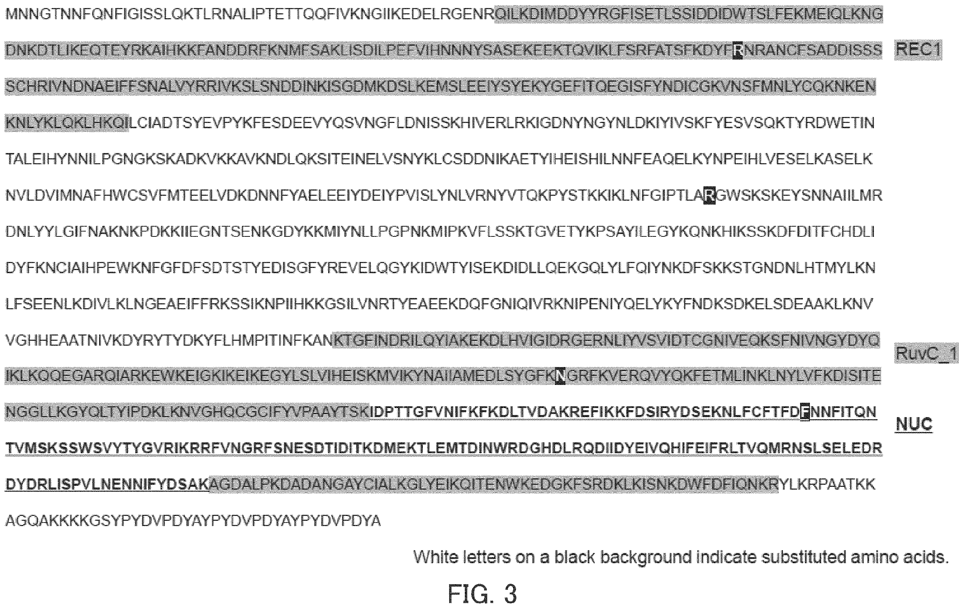

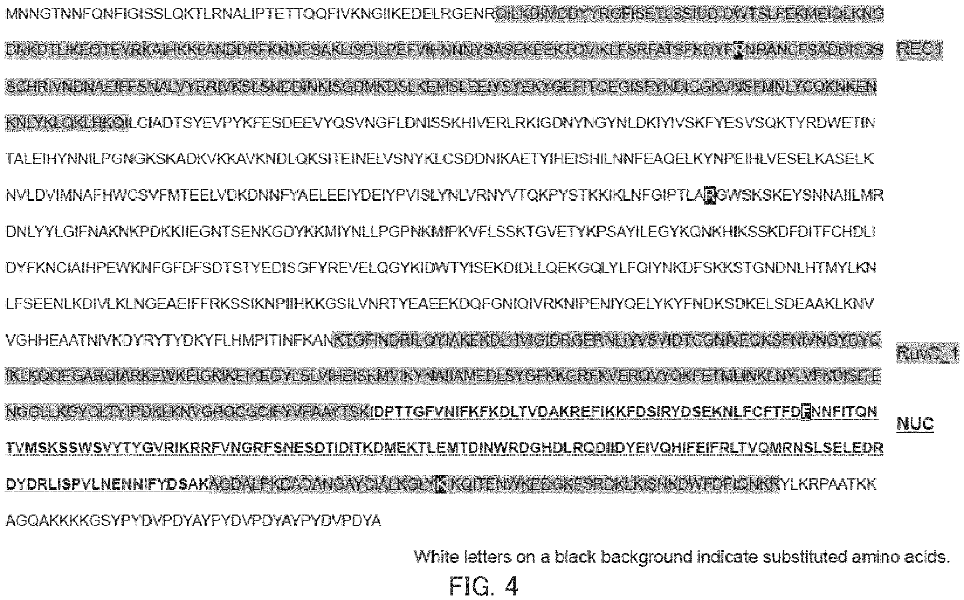

MAD7 expression vectors were obtained by respectively inserting, downstream of the CMV promoter of an expression vector, a coding sequence of a protein (ST7, SEQ ID NO:8: FIGS. 1 and 2) that was an MAD7 protein (SEQ ID NO: 1) in which NLS (SEQ ID NO:4), the GS linker, and the 3×HA tag (SEQ ID NO:5) were added to the C-terminus, a coding sequence of a protein (ST8-1, SEQ ID NO:9: FIGS. 1 and 3) that was a mutant MAD7 protein (ST8-1s, SEQ ID NO:2: a sequence obtained by introducing K169R, D529R, K970N, and Y1086F into SEQ ID NO: 1) in which NLS (SEQ ID NO:4), the GS linker, and the 3 ×HA tag (SEQ ID NO:5) were added to the C-terminus, and a coding sequence of a protein (ST8-2, SEQ ID NO: 10: FIGS. 1 and 4) that was a mutant MAD7 protein (ST8-2s, SEQ ID NO:3: a sequence obtained by introducing K169R, D529R, Y1086F, and E1227K into SEQ ID NO: 1) in which NLS (SEQ ID NO:4), the GS linker, and the 3×HA tag (SEQ ID NO:5) were added to the C-terminus.
Amino acid sequence of NLS (SEQ ID NO:4)
   KRPAAIKKAGQAKKKK
Amino acid sequence of 3 ×HAtag (SEQ ID NO:5)
   YPYDVPDYAYPYDVPDYAYPYDVPDYA
Amino acid sequence of ST7 (SEQ ID NO:8) 
MNNGTNNFQNFIGISSLQKTLRNALIPTETTQQFIVKNGIIKEDELRGENRQILKDIMDDY
YRGFISETLSSIDDIDWTSLFEKMEIQLKNGDNKDTLIKEQTEYRKAIHKKFANDDRFKN
MFSAKLISDILPEFVIHNNNYSASEKEEKTQVIKLFSRFATSFKDYFKNRANCFSADDISSSS
CHRIVNDNAEIFFSNALVYRRIVKSLSNDDINKISGDMKDSLKEMSLEEIYSYEKYGEFITQ
EGISFYNDICGKVNSFMNLYCQKNKENKNLYKLQKLHKQILCIADTSYEVPYKFESDEEV
YQSVNGFLDNISSKHIVERLRKIGDNYNGYNLDKIYIVSKFYESVSQKTYRDWETINTALE
IHYNNILPGNGKSKADKVKKAVKNDLQKSITEINELVSNYKLCSDDNIKAETYIHEISHILN
NFEAQELKYNPEIHLVESELKASELKNVLDVIMNAFHWCSVFMTEELVDKDNNFYAELE
EIYDEIYPVISLYNLVRNYVTQKPYSTKKIKLNFGIPTLADGWSKSKEYSNNAIILMRDNLY
YLGIFNAKNKPDKKIIEGNTSENKGDYKKMIYNLLPGPNKMIPKVFLSSKTGVETYKPSA
YILEGYKQNKHIKSSKDFDITFCHDLIDYFKNCIAIHPEWKNFGFDFSDTSTYEDISGFYRE
VELQGYKIDWTYISEKDIDLLQEKGQLYLFQIYNKDFSKKSTGNDNLHTMYLKNLFSEEN
LKDIVLKLNGEAEIFFRKSSIKNPIIHKKGSILVNRTYEAEEKDQFGNIQIVRKNIPENIYQEL
YKYFNDKSDKELSDEAAKLKNVVGHHEAATNIVKDYRYTYDKYFLHMPITINFKANKT
GFINDRILQYIAKEKDLHVIGIDRGERNLIYVSVIDTCGNIVEQKSFNIVNGYDYQIKLKQQ
EGARQIARKEWKEIGKIKEIKEGYLSLVIHEISKMVIKYNAIIAMEDLSYGFKKGRFKVER
QVYQKFETMLINKLNYLVFKDISITENGGLLKGYQLTYIPDKLKNVGHQCGCIFYVPAAY
TSKIDPTTGFVNIFKFKDLTVDAKREFIKKFDSIRYDSEKNLFCFTFDYNNFITQNTVMSKS
SWSVYTYGVRIKRRFVNGRFSNESDTIDITKDMEKTLEMTDINWRDGHDLRQDIIDYEIV
QHIFEIFRLTVQMRNSLSELEDRDYDRLISPVLNENNIFYDSAKAGDALPKDADANGAYCI
ALKGLYEIKQITENWKEDGKFSRDKLKISNKDWFDFIQNKRYLKRPAATKKAGQAKKKK
GSYPYDVPDYAYPYDVPDYAYPYDVPDYA
Amino acid sequence of ST8-1 (SEQ ID NO:9) 
MNNGTNNFQNFIGISSLQKTLRNALIPTETTQQFIVKNGIIKEDELRGENRQILKDIMDDY
YRGFISETLSSIDDIDWTSLFEKMEIQLKNGDNKDTLIKEQTEYRKAIHKKFANDDRFKN
MFSAKLISDILPEFVIHNNNYSASEKEEKTQVIKLFSRFATSFKDYFRNRANCFSADDISSSS
CHRIVNDNAEIFFSNALVYRRIVKSLSNDDINKISGDMKDSLKEMSLEEIYSYEKYGEFITQ
EGISFYNDICGKVNSFMNLYCQKNKENKNLYKLQKLHKQILCIADTSYEVPYKFESDEEV
YQSVNGFLDNISSKHIVERLRKIGDNYNGYNLDKIYIVSKFYESVSQKTYRDWETINTALE
IHYNNILPGNGKSKADKVKKAVKNDLQKSITEINELVSNYKLCSDDNIKAETYIHEISHILN
NFEAQELKYNPEIHLVESELKASELKNVLDVIMNAFHWCSVFMTEELVDKDNNFYAELE
EIYDEIYPVISLYNLVRNYVTQKPYSTKKIKLNFGIPTLARGWSKSKEYSNNAIILMRDNLY
YLGIFNAKNKPDKKIIEGNTSENKGDYKKMIYNLLPGPNKMIPKVFLSSKTGVETYKPSA
YILEGYKQNKHIKSSKDFDITFCHDLIDYFKNCIAIHPEWKNFGFDFSDTSTYEDISGFYRE
VELQGYKIDWTYISEKDIDLLQEKGQLYLFQIYNKDFSKKSTGNDNLHTMYLKNLFSEEN
LKDIVLKLNGEAEIFFRKSSIKNPIIHKKGSILVNRTYEAEEKDQFGNIQIVRKNIPENIYQEL
YKYFNDKSDKELSDEAAKLKNVVGHHEAATNIVKDYRYTYDKYFLHMPITINFKANKT
GFINDRILQYIAKEKDLHVIGIDRGERNLIYVSVIDTCGNIVEQKSFNIVNGYDYQIKLKQQ
EGARQIARKEWKEIGKIKEIKEGYLSLVIHEISKMVIKYNAIIAMEDLSYGFKNGRFKVER
QVYQKFETMLINKLNYLVFKDISITENGGLLKGYQLTYIPDKLKNVGHQCGCIFYVPAAY
TSKIDPTTGFVNIFKFKDLTVDAKREFIKKFDSIRYDSEKNLFCFTFDFNNFITQNTVMSKS
SWSVYTYGVRIKRRFVNGRFSNESDTIDITKDMEKTLEMTDINWRDGHDLRQDIIDYEIV
QHIFEIFRLTVQMRNSLSELEDRDYDRLISPVLNENNIFYDSAKAGDALPKDADANGAYCI
ALKGLYEIKQITENWKEDGKFSRDKLKISNKDWFDFIQNKRYLKRPAATKKAGQAKKKK
GSYPYDVPDYAYPYDVPDYAYPYDVPDYA
Amino acid sequence of ST8-2 (SEQ ID NO: 10)

MNNGTNNFQNFIGISSLQKTLRNALIPTETTQQFIVKNGIIKEDELRGENRQILKDIMDDY
YRGFISETLSSIDDIDWTSLFEKMEIQLKNGDNKDTLIKEQTEYRKAIHKKFANDDRFKN
MFSAKLISDILPEFVIHNNNYSASEKEEKTQVIKLFSRFATSFKDYFRNRANCFSADDISSSS
CHRIVNDNAEIFFSNALVYRRIVKSLSNDDINKISGDMKDSLKEMSLEEIYSYEKYGEFITQ
EGISFYNDICGKVNSFMNLYCQKNKENKNLYKLQKLHKQILCIADTSYEVPYKFESDEEV
YQSVNGFLDNISSKHIVERLRKIGDNYNGYNLDKIYIVSKFYESVSQKTYRDWETINTALE
IHYNNILPGNGKSKADKVKKAVKNDLQKSITEINELVSNYKLCSDDNIKAETYIHEISHILN

NFEAQELKYNPEIHLVESELKASELKNVLDVIMNAFHWCSVFMTEELVDKDNNFYAELE
EIYDEIYPVISLYNLVRNYVTQKPYSTKKIKLNFGIPTLARGWSKSKEYSNNAIILMRDNLY
YLGIFNAKNKPDKKIIEGNTSENKGDYKKMIYNLLPGPNKMIPKVFLSSKTGVETYKPSA
YILEGYKQNKHIKSSKDFDITFCHDLIDYFKNCIAIHPEWKNFGFDFSDTSTYEDISGFYRE
VELQGYKIDWTYISEKDIDLLQEKGQLYLFQIYNKDFSKKSTGNDNLHTMYLKNLFSEEN
LKDIVLKLNGEAEIFFRKSSIKNPIIHKKGSILVNRTYEAEEKDQFGNIQIVRKNIPENIYQEL
YKYFNDKSDKELSDEAAKLKNVVGHHEAATNIVKDYRYTYDKYFLHMPITINFKANKT
GFINDRILQYIAKEKDLHVIGIDRGERNLIYVSVIDTCGNIVEQKSFNIVNGYDYQIKLKQQ
EGARQIARKEWKEIGKIKEIKEGYLSLVIHEISKMVIKYNAIIAMEDLSYGFKKGRFKVER
QVYQKFETMLINKLNYLVFKDISITENGGLLKGYQLTYIPDKLKNVGHQCGCIFYVPAAY
TSKIDPTTGFVNIFKFKDLTVDAKREFIKKFDSIRYDSEKNLFCFTFDFNNFITQNTVMSKS
SWSVYTYGVRIKRRFVNGRFSNESDTIDITKDMEKTLEMTDINWRDGHDLRQDIIDYEIV
QHIFEIFRLTVQMRNSLSELEDRDYDRLISPVLNENNIFYDSAKAGDALPKDADANGAYCI
ALKGLYKIKQITENWKEDGKFSRDKLKISNKDWFDFIQNKRYLKRPAATKKAGQAKKKK
GSYPYDVPDYAYPYDVPDYAYPYDVPDYA

Also, MAD7 expression vectors were obtained by respectively inserting, downstream of the CMV promoter of an expression vector, coding sequences of proteins in which the NLS (SEQ ID NO:4) and the 3×HA tag (SEQ ID NO:5) were added to the C-termini of sequences obtained by introducing various mutations (the mutation sites are indicated in the diagrams showing the data) into SEQ ID NO: 1.

### Test Example 1-2. Preparation of crRNA Expression Vector

A vector in which the scaffold-crRNA-terminator sequence was inserted downstream of the U6 promoter was prepared.

### Test Example 1-3. Preparation of RFP Expression Vector

A vector in which the red fluorescent protein (RFP) was inserted downstream of the EF-1α promoter was prepared.

### Test Example 1-4. Preparation of EGxxFP Assay Detection Vector

A vector in which the amino group terminal region of EGFP, a cleavable base sequence with the PAM sequence, and the carboxyl group terminal region of EGFP were inserted in this order downstream of the CAG promoter was prepared. This vector was designed such that, when the vector was cleaved through genome editing, the EGFP sequence was recovered and thus green fluorescence was observed.

### Test Example 2. EGxxFP assay

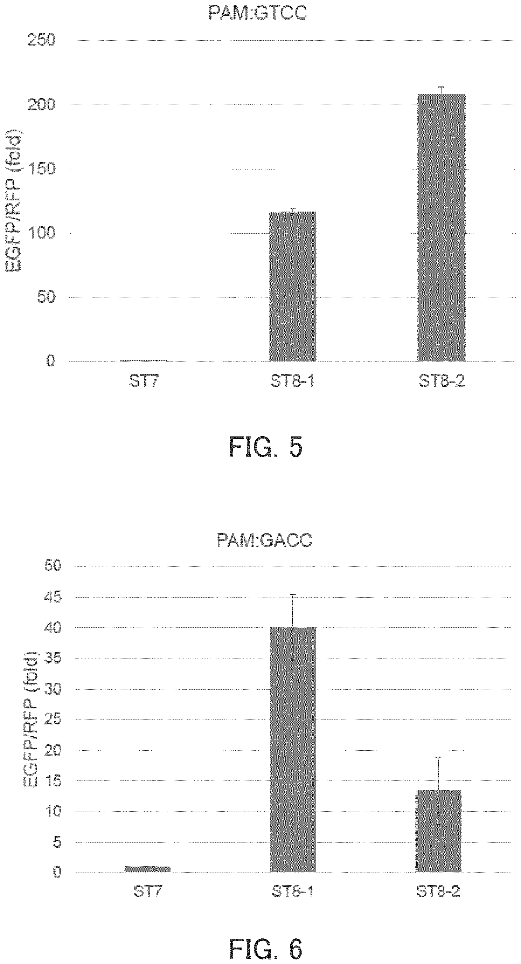

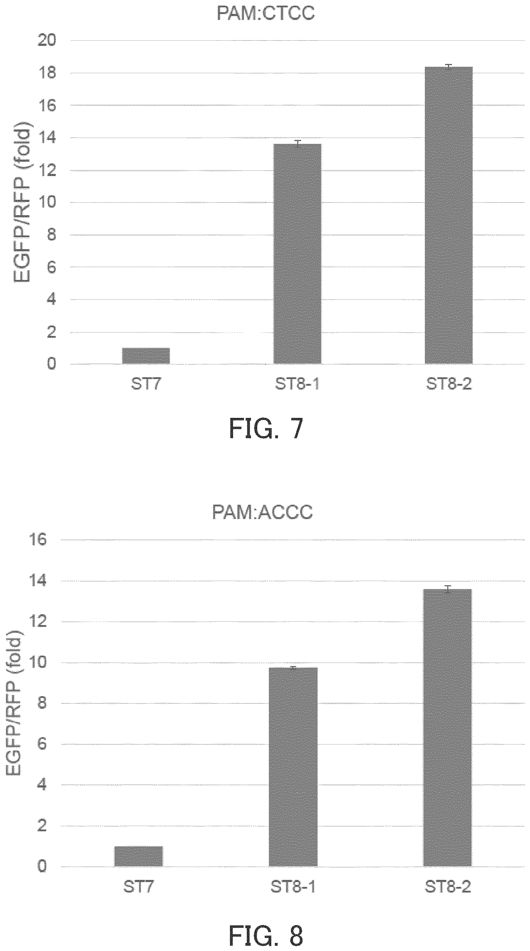

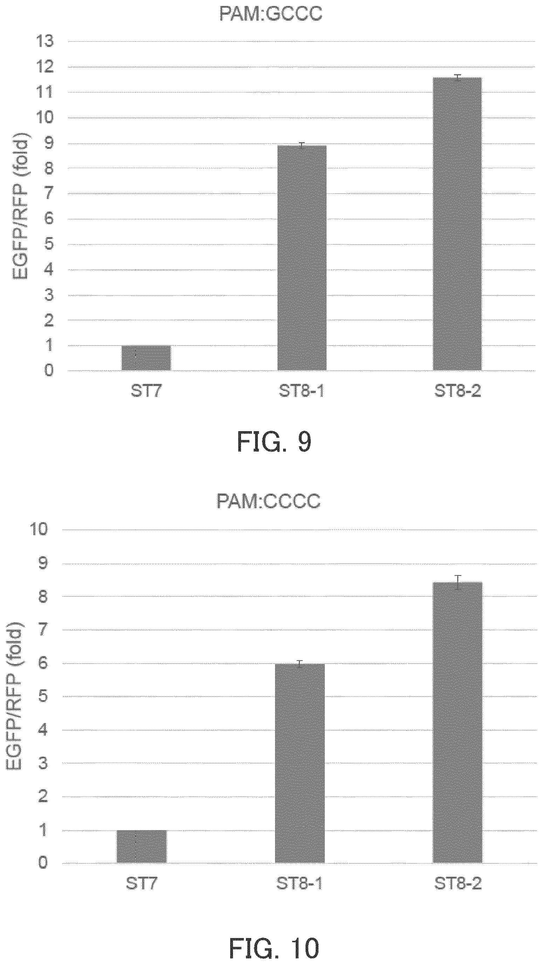

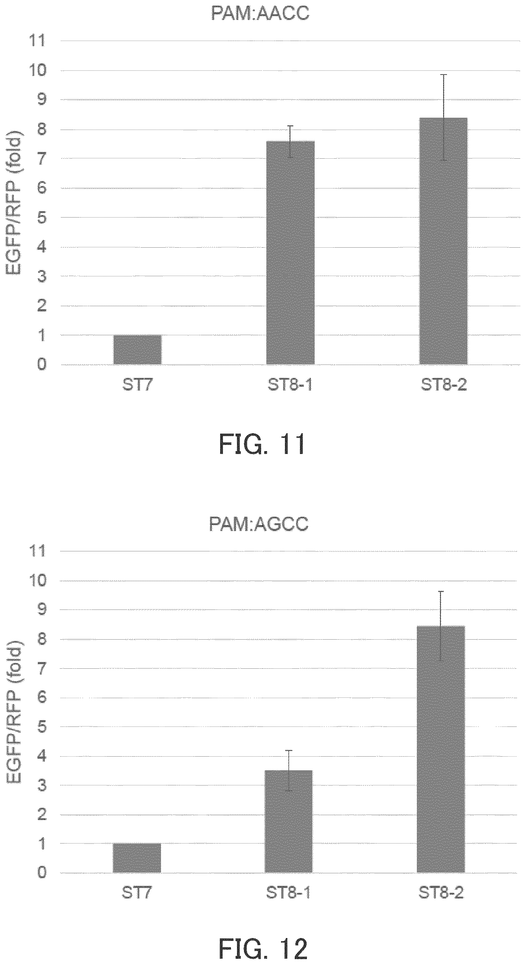

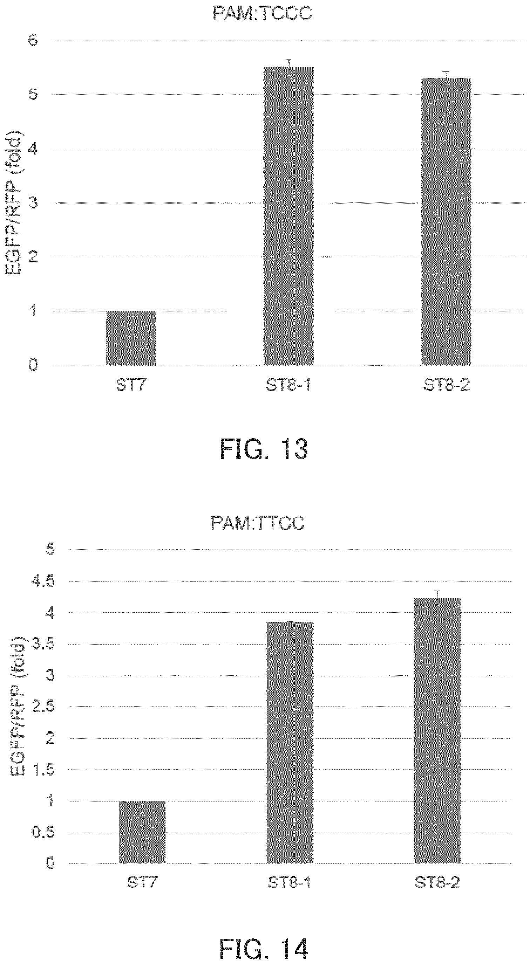

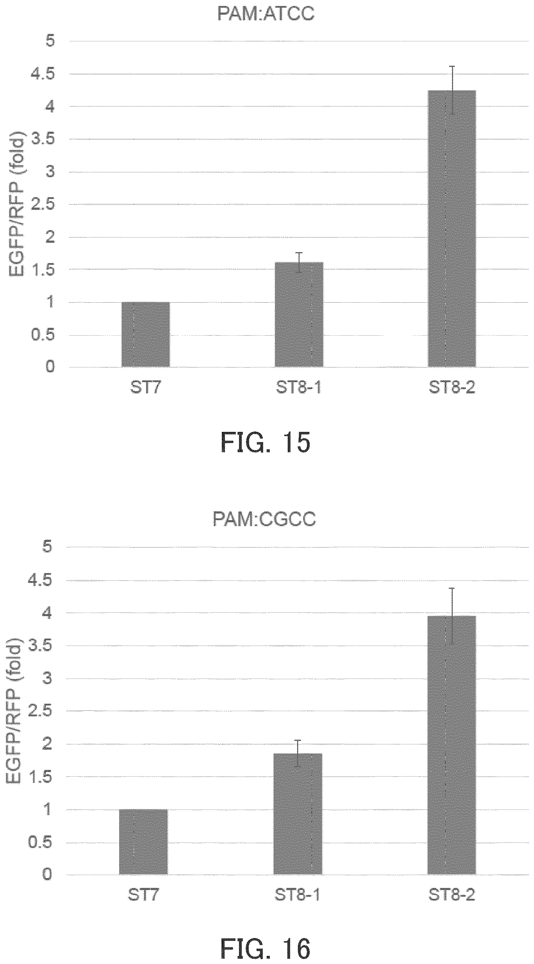

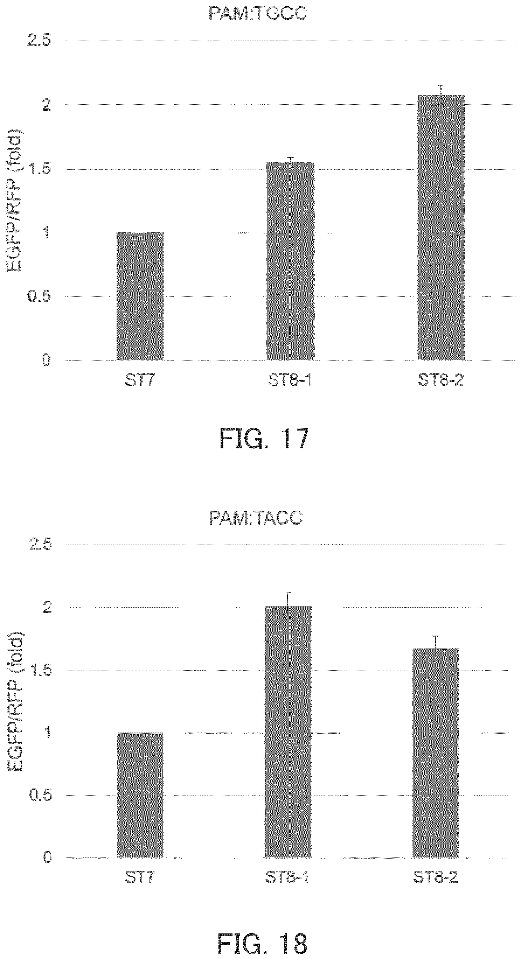

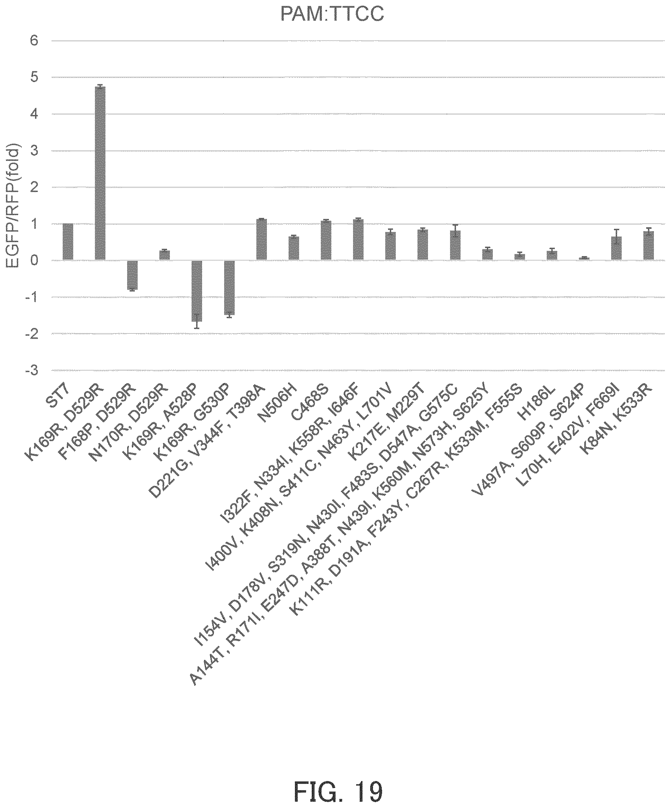

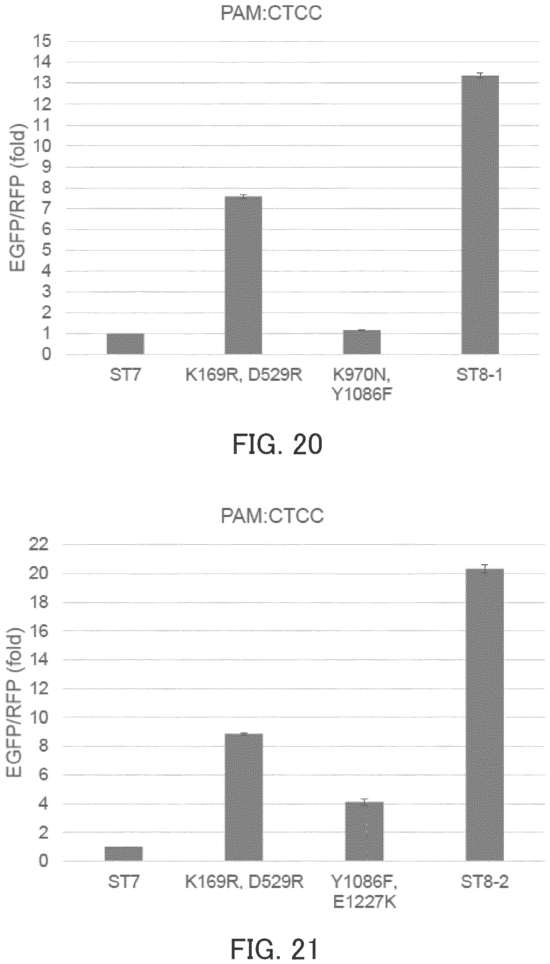

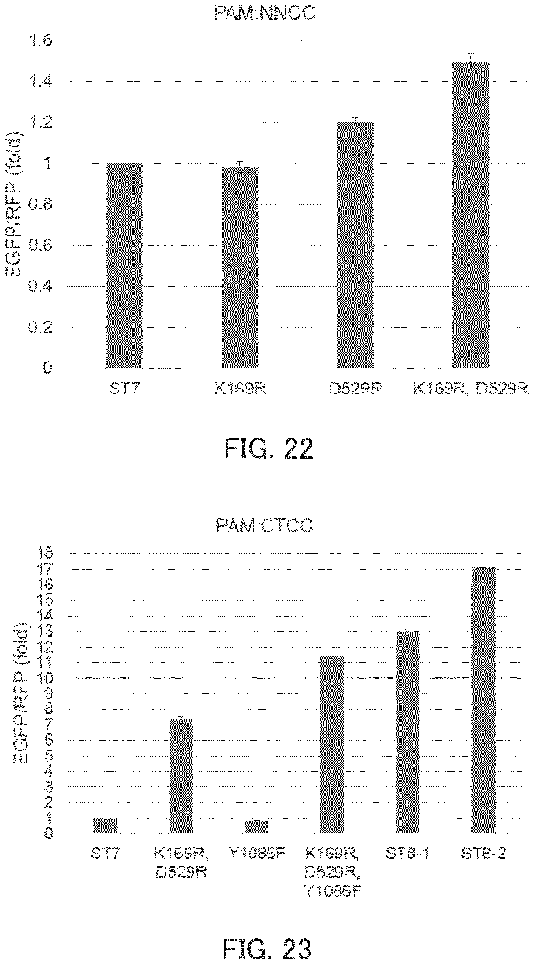

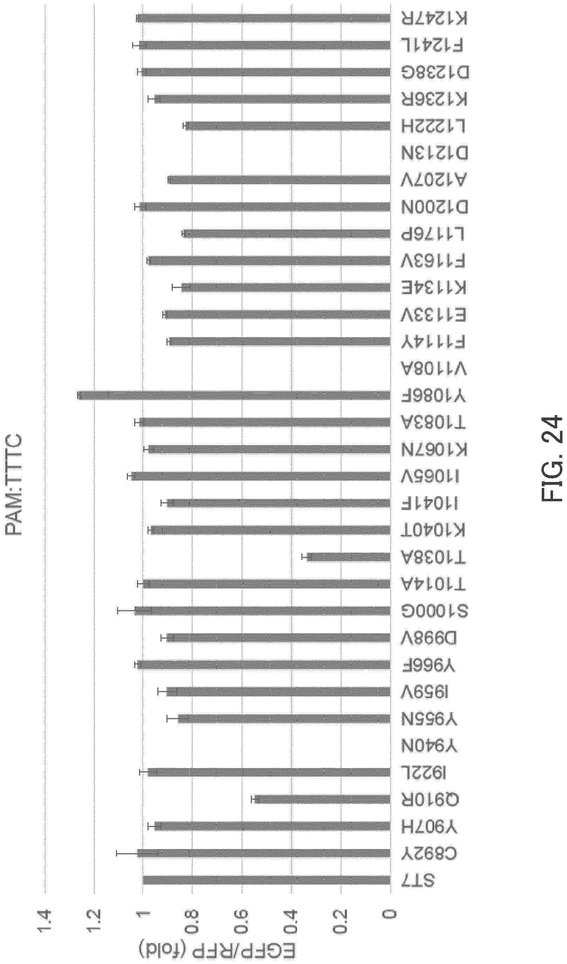

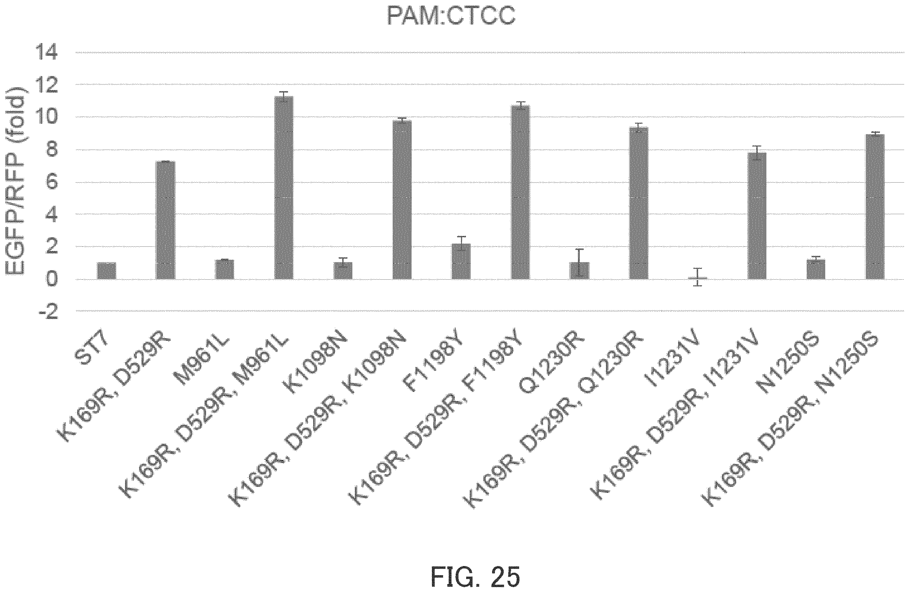

The target-site cleavage activity of each mutant MAD7 protein was measured using the MAD7 expression vectors (Test Example 1). The following describes a specific procedure.
(1) HEK293T cells were seeded in a 24-well plate, and were then cultured in a CO₂ incubator at 37°C for 1.5 days to 2 days. The culture medium was DMEM (High Glucose) (Sigma-Aldrich) supplemented with 10% FBS (CAPRICORN) and 1 × penicillin-streptomycin mixed solution (Nacalai Tesque).
(2) The following mixed solutions were produced.
   Solution A: Opti-MEM (Gibco) 600 µL 2 ng/2 nL RFP expression vector 0.6 µL, 2 ng/2 nL crRNA expression vector 1.2 µL, 2 ng/2 nL EGxxFP assay detection vector 3 µL
   Solution B: Opti-MEM 600 µL, Turbofect (Thermo Fisher Scientific) 24 µL
(3) 75 µL of the solution A was dispensed into tubes, and 0.75 µL of 0.4 ng/nLST7 expression vector or ST8 expression vector was mixed into each tube.
(4) 75 µL of the solution B was added to the solution produced in (3) and mixed well, and the resultant mixture was allowed to stand at room temperature for 20 minutes.
(5) The culture medium in the 24-well plate was replaced with a fresh culture medium.
(6) 50 µL of the solution produced in (4) was added to each well of the 24-well plate, and the plate was mildly shaken.
(7) The cells were cultured for 1.5 days to 2 days, and were then collected through trypsin treatment. A diluted solution of Passive Lysis 5× Buffer (Promega) was added to the cells, and the mixture was vigorously agitated to disrupt the cells.
(8) The mixture was centrifuged at 12000 rpm for 5 minutes, and the supernatant was collected. 50 µL of the supernatant of each sample was added to two wells of a 96-well plate, and the fluorescent intensities of EGFP and RFP contained in the supernatant were detected using a plate reader (CYTATION3 imaging reader (BioTek)).
(9) The EGFP value was normalized by the RFP value. A sample containing no MAD7 expression vector was used as a negative control, and the value from the negative control was subtracted from the value from each sample. The value from ST7 was taken as 1, and the values from the other samples were indicated as values relative to ST7. Each of the data was obtained from three replicates, and the error range was indicated as a standard error. FIGS. 5 to 25 show the results.

### Test Example 3. Production of Mutant MAD7 Protein

The MAD7 and mutant MAD7 proteins were purified using the E. coli protein expression system, pET system. The following describes a specific procedure.
(1) A pET expression vector that included the coding sequence of the MAD7 or mutant MAD7 (Test Example 1) was used to transform Rosetta (trademark) (DE3) Competent cells (Sigma-Aldrich), and the cells were cultured on an LB agar culture medium containing kanamycin sulfate. The cells were incubated at 37°C for 1 day, and the formation of E. coli colonies was confirmed.
(2) One colony was selected from the formed colonies, and was then cultured in 10 mL of an LB liquid culture medium containing kanamycin sulfate at 37°C at 180 rpm for 6 hours (preculture).
(3) 1 mL of the precultured LB culture solution and 100 mL of an LB liquid culture medium containing kanamycin sulfate was placed in each of ten baffled Erlenmeyer flasks, and was then incubated at 37°C at 180 rpm for 8 hours.
(4) The culture medium was allowed to stand on ice for 15 minutes, and the IPTG (Merck) was added thereto to give a final concentration of 0.5 mM. Then, the resultant mixture was incubated at 25°C at 130 rpm for 6 hours.
(5) The mixture was centrifuged at 4°C at 3000g for 10 minutes. The supernatant was removed, and the obtained E. coli pellet was washed using PBS.
(6) The E. coli pellet was subjected to lysozyme treatment and supersonic treatment, and was centrifuged at 4°C at 70000×g for 30 minutes. Then, a soluble fraction was collected.
(7) The soluble fraction was separated and purified through column chromatography using NGC (trademark) Chromatography System (Bio-Rad).
(8) The separated and purified MAD7 and mutant MAD7 were electrophoresed on SDS-PAGE and confirmed through CBB staining (Wako).

### Test Example 4. In Vitro Assay

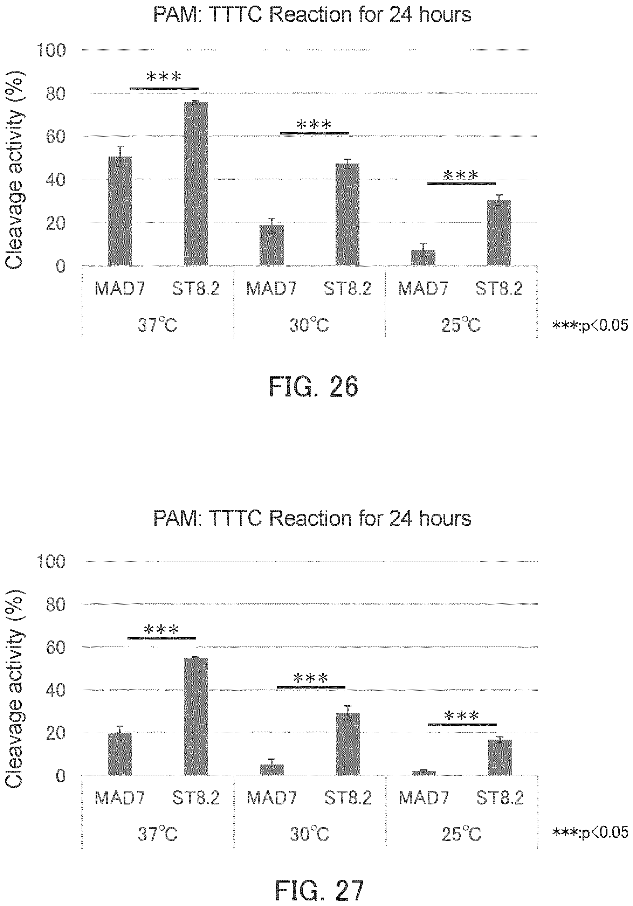

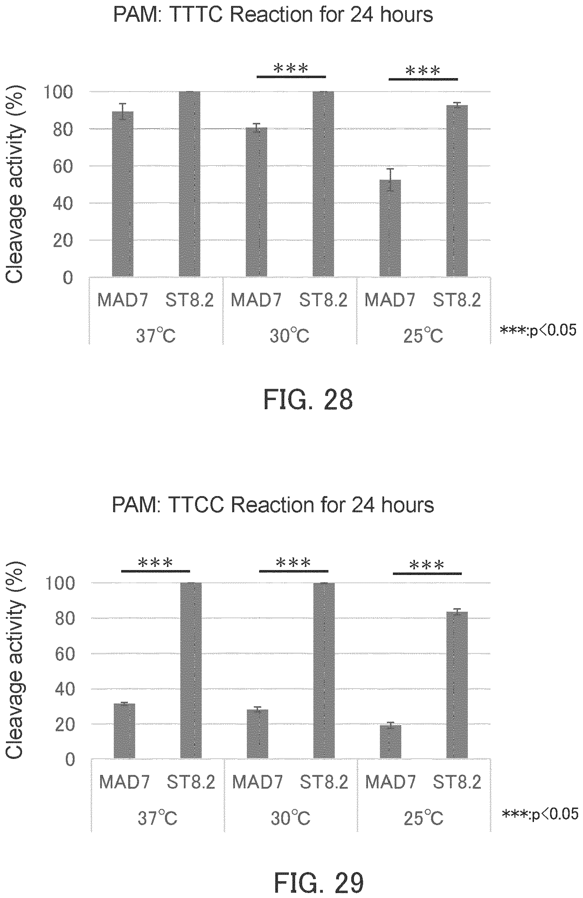

(1) The amount ratio between the MAD7 protein (Test Example 3) and the ST8.2 protein (Test Example 3) was calculated through CBB staining.
(2) 10 µL of each of the MAD7 protein solution and the ST8.2 protein solution of the same concentration was mixed with 2 µL of NE Buffer 3.1, 3 µL of crRNA [sequence: /AlTR1/rUrArArUrUrUrCrUrArCrUrCrUrUrGrUrArGrArUrUrGrUrCrArCrCrArArUrCrCrUrGrU rCrCrCrUrArG (SEQ ID NO:6)/AlTR2/] (100 ng/µL), and sterilized water, and the resultant mixture was allowed to stand at 25°C for 10 minutes.
(3) 500 ng of a substrate DNA was added, and the volume of the reaction solution was set to 20 µl.
(4) The reaction temperature was 37°C, 30°C, or 25°C, and the reaction time was 1 hour or 24 hours.
(5) 4 µL of the 10× loading buffer was added.
(6) 10 µL of each of the reaction solutions was electrophoresed on 2% agarose gel.
(7) A photograph of the gel was taken using a gel imaging apparatus, and the amount of the DNA for cleavage that had not been cleaved was measured using ImageJ.
(8) The cleavage activity was calculated based on the following calculation formula: 100×((amount of control substrate DNA - amount of uncleaved substrate DNA) / amount of control substrate DNA). FIGS. 26 to 29 show the results.

### Test Example 5. Analysis of Mutagenesis Efficiency in Mouse Fertilized Egg

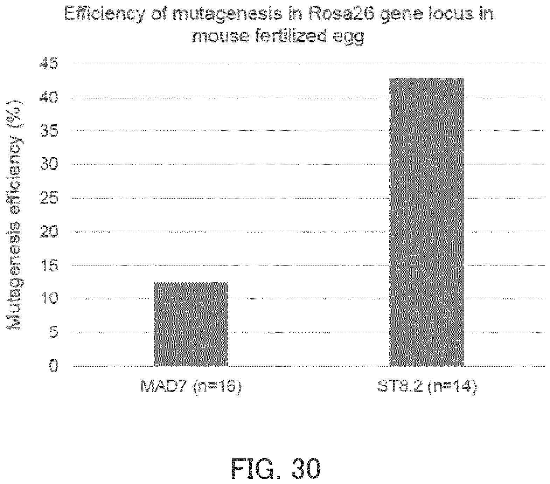

(1) Each of the MAD7 and the ST8.2 of the same concentration and crRNA [sequence: /AlTR1/rUrArArUrUrUrCrUrArCrUrCrUrUrGrUrArGrArUrCrArCrCrUrGrUrUrCrArArUrUrCrC rCrCrUrGrCrA/AlTR2/] (SEQ ID NO:7) designed to be targeted on the mouse Rosa26 gene locus were inserted into a mouse fertilized egg using the GEEP method (JP 2017-184639A).
(2) The electroporated fertilized eggs were cultured in a CO₂ incubator at 37°C until E3.5 to 4.5, and the fertilized eggs that had grown to a morula or blastocyst were individually collected.
(3) DNA was extracted from each of the collected fertilized eggs.
(4) PCR was performed using primers for detecting the target sequence of the crRNA designed to be targeted on the Rosa26 gene locus.
(5) DNA sequence analysis was performed using Miseq reagent nano kit v2.
(6) A mutation caused by cleavage of the genome DNA was confirmed in the Rosa26 gene locus.
(7) The cleavage efficiency was calculated based on the following calculation formula: 100×(number of specimens in which mutation caused by cleavage of genome DNA was detected in Rosa26 gene locus / total number of specimens). FIG. 30 shows the results.

As described above, the present invention has been described with reference to the embodiment, but the present invention is not limited to the above-described embodiment. Various modifications that can be understood by a person skilled in the art can be made in the configurations and details of the present invention without departing from the scope of the present invention.

The present application claims the benefit of priority from Japanese Patent Application No. 2022-100914 filed on January 28, 2022, the entire disclosure of which is incorporated herein.

[Sequence Listing]

## Claims

1. A mutant MAD7 protein comprising an amino acid sequence B, the mutant MAD7 protein having mutations in an amino acid sequence A represented by SEQ ID NO: 1, the amino acid sequence B comprising amino-acid substitution mutations of K169 and D529 in the amino acid sequence A.

2. The mutant MAD7 protein according to claim 1, wherein the amino acid sequence B comprises an amino acid sequence of (a1), (a2), or (a3) below:
(a1) an amino acid sequence that comprises amino-acid substitution mutations of K169 and D529 in the amino acid sequence represented by SEQ ID NO: 1;
(a2) an amino acid sequence that has at least one mutation selected from the group consisting of a deletion, an insertion, a substitution, and an addition between a first and a 126th amino acids, inclusive, in the amino acid sequence represented by SEQ ID NO: 1 and comprises the amino-acid substitution mutations of (a1) above; and
(a3) an amino acid sequence that has 90% or more identity to the amino acid sequence represented by SEQ ID NO: 1 and comprises the amino-acid substitution mutations of (a1) above.

3. The mutant MAD7 protein according to claim 1 or 2, wherein the amino-acid substitution mutations are a substitution of K169 with a basic amino acid other than lysine, and a substitution of D529 with a basic amino acid.

4. The mutant MAD7 protein according to any one of claims 1 to 3, wherein the amino-acid substitution mutations are a substitution of K169 with arginine, and a substitution of D529 with arginine.

5. The mutant MAD7 protein according to any one of claims 1 to 4, wherein the amino acid sequence B comprises an amino-acid substitution mutation of Y1086 in the amino acid sequence A.

6. The mutant MAD7 protein according to claim 5, wherein the amino-acid substitution mutation is a substitution ofY1086 with an aromatic amino acid other than tyrosine.

7. The mutant MAD7 protein according to claim 5 or 6, wherein the amino-acid substitution mutation is a substitution ofY1086 with phenylalanine.

8. The mutant MAD7 protein according to any one of claims 1 to 7, wherein the amino acid sequence B comprises in the amino acid sequence A, an amino-acid substitution mutation of at least one amino acid selected from the group consisting of K970 and E1227.

9. The mutant MAD7 protein according to claim 8, wherein the amino-acid substitution mutation is at least one mutation selected from the group consisting of a substitution of K970 with a hydrophilic neutral amino acid and a substitution of E1227 with a basic amino acid.

10. The mutant MAD7 protein according to claim 8 or 9, wherein the amino-acid substitution mutation is at least one mutation selected from the group consisting of a substitution of K970 with asparagine and a substitution of E1227 with lysine.

11. The mutant MAD7 protein according to any one of claims 1 to 10, wherein the amino acid sequence B comprises in the amino acid sequence A, an amino acid substitution mutation of at least one amino acid selected from the group consisting of M961, K1098, F1198, Q1230, 11231, and N1250.

12. The mutant MAD7 protein according to any one of claims 1 to 11, wherein the amino acid sequence B comprises in the amino acid sequence A, an amino-acid substitution mutation of at least one amino acid selected from the group consisting of C892, Y907, I922, Y966, S1000, T1014, K1040, L1056, I1065,K1067, T1083, F1163, D1200, K1236, D1238, F1241, S1242, and K1247.

13. The mutant MAD7 protein according to any one of claims 1 to 12, wherein the amino acid sequence B comprises an amino acid sequence of (b1), (b2), or (b3) below:
(b1) an amino acid sequence represented by SEQ ID NO:2;
(b2) an amino acid sequence that has at least one mutation selected from the group consisting of a deletion, an insertion, a substitution, and an addition between a first and a 126th amino acids, inclusive, in the amino acid sequence represented by SEQ ID NO:2 and in which amino acids at position 169 and position 529 in the amino acid sequence represented by SEQ ID NO:2 are conserved; and
(b3) an amino acid sequence that has 90% or more identity to the amino acid sequence represented by SEQ ID NO:2 and in which amino acids at position 169 and position 529 in the amino acid sequence represented by SEQ ID NO:2 are conserved.

14. The mutant MAD7 protein according to claim 13, wherein in (b2) or (b3) above, an amino acid at position 1086 in the amino acid sequence represented by SEQ ID NO:2 is further conserved.

15. The mutant MAD7 protein according to claim 13 or 14, wherein in (b2) or (b3) above, at least one amino acid selected from the group consisting of an amino acid at position 970 and an amino acid at position 1227 in the amino acid sequence represented by SEQ ID NO:2 is further conserved.

16. The mutant MAD7 protein according to any one of claims 1 to 12, wherein the amino acid sequence B comprises an amino acid sequence of (c1), (c2), or (c3) below:
(c1) an amino acid sequence represented by SEQ ID NO:3;
(c2) an amino acid sequence that has at least one mutation selected from the group consisting of a deletion, an insertion, a substitution, and addition between a first and a 126th amino acids, inclusive, in the amino acid sequence represented by SEQ ID NO:3 and in which amino acids at position 169 and position 529 in the amino acid sequence represented by SEQ ID NO:3 are conserved; and
(c3) an amino acid sequence that has 90% or more identity to the amino acid sequence represented by SEQ ID NO:3 and in which amino acids at position 169 and position 529 in the amino acid sequence represented by SEQ ID NO:3 are conserved.

17. The mutant MAD7 protein according to claim 13 or 14, wherein, in (c2) or (c3) above, an amino acid at position 1086 in the amino acid sequence represented by SEQ ID NO:3 is further conserved.

18. The mutant MAD7 protein according to claim 16 or 17, wherein, in (c2) or (c3) above, at least one amino acid selected from the group consisting of an amino acid at position 970 and an amino acid at position 1227 in the amino acid sequence represented by SEQ ID NO:3 is further conserved.

19. The mutant MAD7 protein according to any one of claims 1 to 18, comprising the amino acid sequence B and a nuclear localization signal sequence.

20. A combination of polypeptides, comprising a first split fragment and a second split fragment of the mutant MAD7 protein according to any one of claims 1 to 18,
wherein the first split fragment and the second split fragment are polypeptides having amino acid sequences selected from the group consisting of (1) to (10) and (11) below:
(1) the first split fragment has an amino acid sequence corresponding to an amino acid sequence between positions 1 and 182 in SEQ ID NO: 1, and the second split fragment has an amino acid sequence corresponding to an amino acid sequence between positions 183 and 1263 in SEQ ID NO:1;
(2) the first split fragment has an amino acid sequence corresponding to an amino acid sequence between positions 1 and 273 in SEQ ID NO: 1, and the second split fragment has an amino acid sequence corresponding to an amino acid sequence between positions 274 and 1263 in SEQ ID NO:1;
(3) the first split fragment has an amino acid sequence corresponding to an amino acid sequence between positions 1 and 292 in SEQ ID NO: 1, and the second split fragment has an amino acid sequence corresponding to an amino acid sequence between positions 293 and 1263 in SEQ ID NO:1;
(4) the first split fragment has an amino acid sequence corresponding to an amino acid sequence between positions 1 and 377 in SEQ ID NO: 1, and the second split fragment has an amino acid sequence corresponding to an amino acid sequence between positions 378 and 1263 in SEQ ID NO:1;
(5) the first split fragment has an amino acid sequence corresponding to an amino acid sequence between positions 1 and 511 in SEQ ID NO: 1, and the second split fragment has an amino acid sequence corresponding to an amino acid sequence between positions 512 and 1263 in SEQ ID NO:1;
(6) the first split fragment has an amino acid sequence corresponding to an amino acid sequence between positions 1 and 538 in SEQ ID NO: 1, and the second split fragment has an amino acid sequence corresponding to an amino acid sequence between positions 539 and 1263 in SEQ ID NO:1;
(7) the first split fragment has an amino acid sequence corresponding to an amino acid sequence between positions 1 and 567 in SEQ ID NO: 1, and the second split fragment has an amino acid sequence corresponding to an amino acid sequence between positions 568 and 1263 in SEQ ID NO:1;
(8) the first split fragment has an amino acid sequence corresponding to an amino acid sequence between positions 1 and 755 in SEQ ID NO: 1, and the second split fragment has an amino acid sequence corresponding to an amino acid sequence between positions 756 and 1263 in SEQ ID NO:1;
(9) the first split fragment has an amino acid sequence corresponding to an amino acid sequence between positions 1 and 832 in SEQ ID NO: 1, and the second split fragment has an amino acid sequence corresponding to an amino acid sequence between positions 833 and 1263 in SEQ ID NO:1;
(10) the first split fragment has an amino acid sequence corresponding to an amino acid sequence between positions 1 and 854 in SEQ ID NO: 1, and the second split fragment has an amino acid sequence corresponding to an amino acid sequence between positions 855 and 1263 in SEQ ID NO: 1; and
(11) the first split fragment has an amino acid sequence corresponding to an amino acid sequence between positions 1 and 49 and between positions 512 and 1263 in SEQ ID NO: 1 in which an amino acid Y corresponding to position 513 in SEQ ID NO: 1 is substituted with S, and the second split fragment has an amino acid sequence corresponding to an amino acid sequence between positions 50 and 511 in SEQ ID NO: 1.

21. A polynucleotide comprising a coding sequence of the mutant MAD7 protein according to any one of claims 1 to 19 and/or coding sequences of the polypeptides according to claim 20.

22. A vector comprising the polynucleotide according to claim 21.

23. A composition comprising the mutant MAD7 protein according to any one of claims 1 to 19, the combination of polypeptides according to claim 20, the polynucleotide according to claim 21, and/or the vector according to claim 22, and a guide RNA targeted on a target site.

24. A cell comprising the mutant MAD7 protein according to any one of claims 1 to 19, the combination of polypeptides according to claim 20, the polynucleotide according to claim 21, and/or the vector according to claim 22.

25. A genome editing method comprising introducing, into a cell or non-human organism, the mutant MAD7 protein according to any one of claims 1 to 19, the combination of polypeptides according to claim 20, the polynucleotide according to claim 21, and/or the vector according to claim 22.

26. The genome editing method according to claim 25, further comprising introducing a guide RNA targeted on a target site into the cell or non-human organism.

27. A method for producing a cell or organism in which a target site is genome-edited, comprising
introducing into a cell or non-human organism, the mutant MAD7 protein according to any one of claims 1 to 19, the combination of polypeptides according to claim 20, the polynucleotide according to claim 21, and/or the vector according to claim 22, and the guide RNA targeted on a target site.

28. A cell or organism obtained using the method for producing a cell or organism according to claim 27.

29. A genome editing composition for experimental use comprising the mutant MAD7 protein according to any one of claims 1 to 19, the combination of polypeptides according to claim 20, the polynucleotide according to claim 21, the vector according to claim 22, and/or the cell according to claim 24.

30. A genome editing composition for agricultural use comprising the mutant MAD7 protein according to any one of claims 1 to 19, the combination of polypeptides according to claim 20, the polynucleotide according to claim 21, the vector according to claim 22, and/or the cell according to claim 24.

31. A genome editing composition for medical use comprising the mutant MAD7 protein according to any one of claims 1 to 19, the combination of polypeptides according to claim 20, the polynucleotide according to claim 21, the vector according to claim 22, and/or the cell according to claim 24.

32. A genome editing composition for livestock use comprising the mutant MAD7 protein according to any one of claims 1 to 19, the combination of polypeptides according to claim 20, the polynucleotide according to claim 21, the vector according to claim 22, and/or the cell according to claim 24.

33. A genome editing composition for fishery use comprising the mutant MAD7 protein according to any one of claims 1 to 19, the combination of polypeptides according to claim 20, the polynucleotide according to claim 21, the vector according to claim 22, and/or the cell according to claim 24.

34. A genome editing composition for industrial use comprising the mutant MAD7 protein according to any one of claims 1 to 19, the combination of polypeptides according to claim 20, the polynucleotide according to claim 21, the vector according to claim 22, and/or the cell according to claim 24.
